# EUROPEAN PATENT SPECIFICATION

(11) **EP 2 087 351 B1**
(45) Date of publication and mention of the grant of the patent: **21.11.2012**
(21) Application number: 07867222.7
(22) Date of filing: 11.10.2007
(51) Int. Cl.: G01N 33/53, G01N 33/566, C07K 14/705

(54) **METHODS TO UTILIZE INVERTEBRATE CHEMOSENSORY PROTEINS FOR INDUSTRIAL AND COMMERCIAL USES**
VERFAHREN ZUR VERWENDUNG CHEMOSENSORISCHER INVERTEBRATENPROTEINE FÜR INDUSTRIELLE UND KOMMERZIELLE ZWECKE
PROCÉDÉS POUR UTILISER DES PROTÉINES CHIMIOSENSIBLES D'INVERTÉBRÉS POUR DES APPLICATIONS INDUSTRIELLES ET COMMERCIALES

(30) Priority: 16.10.2006 US 581889
(43) Date of publication of application: 12.08.2009
(73) Proprietor: Woods, Daniel, F., Irvine, CA 92614 (US); Dimitratos, Spiros, D., Irvine, CA 92614 (US); Justice, Robin, W., Irvine, CA 92614 (US)
(72) Inventor: Woods, Daniel, F., Irvine, CA 92614 (US); Dimitratos, Spiros, D., Irvine, CA 92614 (US); Justice, Robin, W., Irvine, CA 92614 (US)
(74) Representative: Novagraaf Technologies
(86) International application number: PCT/US2007/021820
(87) International publication number: WO 2008/091306

(56) References cited:
- WO-A2-02/077200
- US-A- 5 981 195
- US-A1- 2004 003 419
- SUNG J H ET AL: "Piezoelectric biosensor using olfactory receptor protein expressed in Escherichia coli" BIOSENSORS AND BIOELECTRONICS, ELSEVIER BV, NL, vol. 21, no. 10, 15 April 2006 (2006-04-15), pages 1981-1986, XP024961674 ISSN: 0956-5663 [retrieved on 2006-04-15]
- FENG ET AL.: 'Expression and Characterization of a Lepidopteran General Odorant Binding Protein' INSECT BIOCHEMISTRY AND MOLECULAR BIOLOGY vol. 27, no. 5, 1997, pages 405 - 412, XP008109146
- VOGT ET AL.: 'Odorant Binding Protein Diversity and Distribution among the Isect Orders, as Indicated by LAP, an OBP-related Protein of the True Bug Lygus lineolaris (Hemiptera, Heteroptera)' CHEMICAL SENSES vol. 24, no. 5, October 1999, pages 481 - 494, XP008109173

## Description

### Technical Field

The present invention generally relates to methods and compositions for utilizing chemosensory proteins sourced from invertebrates in detectors, sensors, biosensors, and other sensor devices. As part of this set of applications, the invention also relates to methods for utilizing chemosensory proteins in collections or arrays in order to identify specific chemosensory cues or analytes to be detected. Furthermore, the invention describes the methods and means necessary to construct purification devices capable of detecting, isolating and purifying an analyte of interest from a complex mixture in the atmosphere or in solution. The technologies presented are feasible in a variety of applications where detection of an analyte or the absence of an analyte is desirable.

This invention can be incorporated into biosensors or other devices used for industrial, domestic, food safety, pharmaceutical, military, security, purification, and other applications.

### Background

The Electronic Nose is a chemical sensor system that detects volatile compounds or analytes. Electronic Nose technology is being utilized or developed for a wide variety of applications, from medical diagnostics to environmental monitoring.¹⁻⁵ For example, the development of inexpensive, reliable detectors for the presence of volatile organic compounds (VOCs) is a particularly acute issue. Volatile organic compounds are emitted as gases from certain solids or liquids; VOCs include chemicals that have short- and long-term adverse health effects and/or are environmental risks, and their concentrations are consistently higher indoors (up to five times higher) than outdoors. Potentially harmful chemicals are emitted by a wide array of products numbering in the thousands (paints and lacquers, paint strippers, cleaning supplies, pesticides, building materials and furnishings, office equipment such as copiers and printers, correction fluids and carbonless copy paper, graphics and craft materials including glues and adhesives, permanent markers, photographic solutions, *etc*.). VOCs have also been demonstrated to be reliable markers of human diseases.⁶⁻¹³

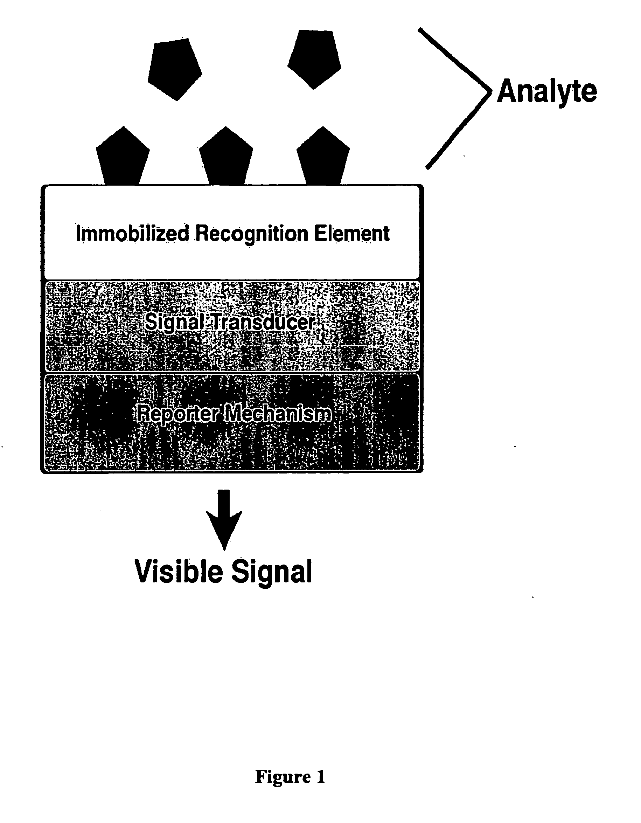

A key limitation of the present generation of chemical sensors is the recognition elements that bind the compound being detected (Figure 1). The recognition elements currently used in electronic noses are based primarily on conducting polymer recognition elements. Their limitations include sensor drift (no absolute calibration), limited sensitivity and selectivity, sensor poisoning (sensitivity to a high concentration of certain analytes), lack of relationship between odor quality and intensity, sensitivity to water or other polar compounds (*e.g*., EtOH), useful lifespan limitations, and high cost.³ Often the fluctuation in performance leads to difficulties reproducing data. Another major limitation of current sensor technology is the failure of available sensors in distinguishing between structurally related compounds.¹⁴⁻¹⁶ Sensors based on artificial polymer recognition elements are also generally limited in their ability to identify specific analytes in complex mixtures.^{15,17,18} Thus, sensor technology can greatly benefit from inexpensive, practical, high sensitivity recognition elements capable of overcoming several of the current limitations.

Biosensors are analytical tools that measure the presence of a single molecular species in complex mixtures by combining the exquisite molecular recognition properties of biological macromolecules with the generation of a detectable signal. Thus, biosensors overcome a critical drawback of the previously mentioned, non-biological sensor technologies - lack of analyte specificity. To date biosensors have incorporated enzymes or antibodies.¹⁹⁻²²

Method for isolating or identifying molecules, chemicals or reagents, that interact with isolated odorant receptors, for example GPCRs, OBPs, or other olfactory proteins, is disclosed in the art (International Application WO 02/077200).

Isolated nucleic acid molecules encoding amino acid sequences of Drosophila gustatory receptor proteins for the identification of binding partners (explosives, drugs) and for generation of fingerprints of chemicals using appropriate marker or chemical or electric signals, are disclosed in the art. The protein may be a variant including Diptera, Lepidoptera, Homopterera and Coleoptera, within these orders, preferably the genus Drosophila, Anopheles, Aedes, Ceratitis, Muscidae, Culicidae, Anagasta and Popilla (U.S. Application 2004/003419).

The expression of the olfactory receptor protein of *C*. *elegans,* ODR-10, on the membrane of *Escherichia coli,* was used to detect odorants molecules, thereby creating a characteristic profile (Sung et al., Biosensors and Bioelectronics, Elsevier BV, NL, 21(10) : 1981-1986, 2006).

This invention provides the means and methods necessary to overcome current sensor limitations by utilizing the diversity, versatility and specificity of the biomolecules comprising the invertebrate chemosensory system; this includes the olfactory and gustatory systems that comprise similar proteins and effectors.²³ The invention also relates to the selective purification, identification, or exclusion of a desired or undesired analyte from a mixture, solution, or the atmosphere.

The rich molecular diversity present in insect chemosensory systems is an untapped yet valuable resource with applications in a wide range of areas including pharmaceutical screening, medical diagnostics, and chemical detection.^{4,5,24,25} The insect chemosensory machinery can be adapted to construct biosensor devices capable of detecting and isolating environmental contaminants or impurities in industrial processes, of detecting compounds or molecules in the environment or water and of screening pharmaceutical candidates. Additional applications for efficient compound detectors include the detection of diagnostic breath components in patients with cancer and other diseases_{.}^{5,6,24}

### Brief Description of the Figures

**Figure 1****. Parts of a modern chemical sensor.** Several well-developed mechanisms exist for both the signal transducer and reporter mechanism including those based on metal oxide (for example, MOSFET: metal oxide semiconductor field effect transistor or CMOS: complementary metal oxide semiconductor)¹⁸ and piezoelectric devices,^{18,106,107} however the recognition elements suffer from several limitations (Table 1).

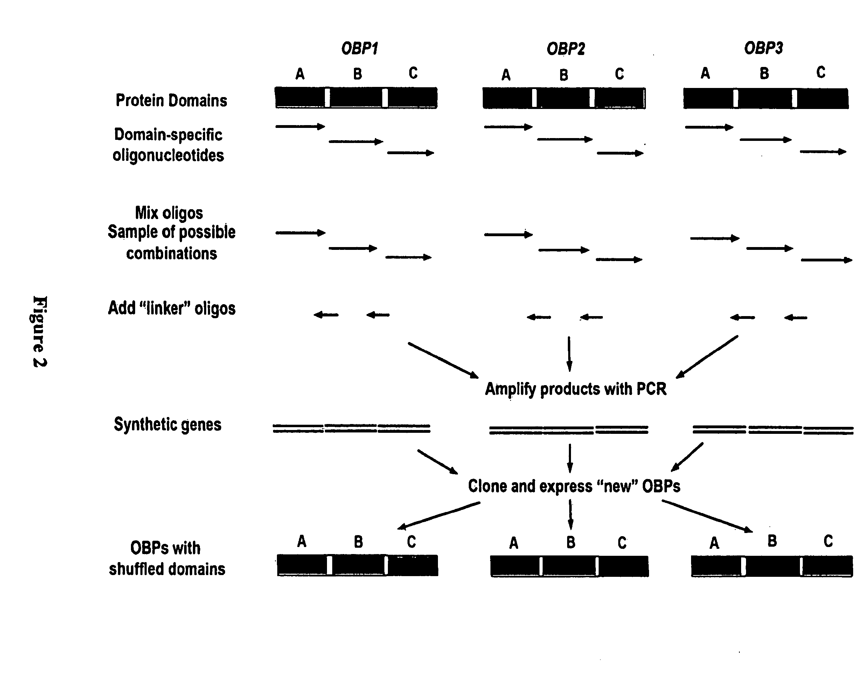

**Figure 2****. Generating chemosensory proteins with novel ligand binding domains.** These hybrid chemosensory proteins can then be used in chemosensory arrays to recognize novel analytes.

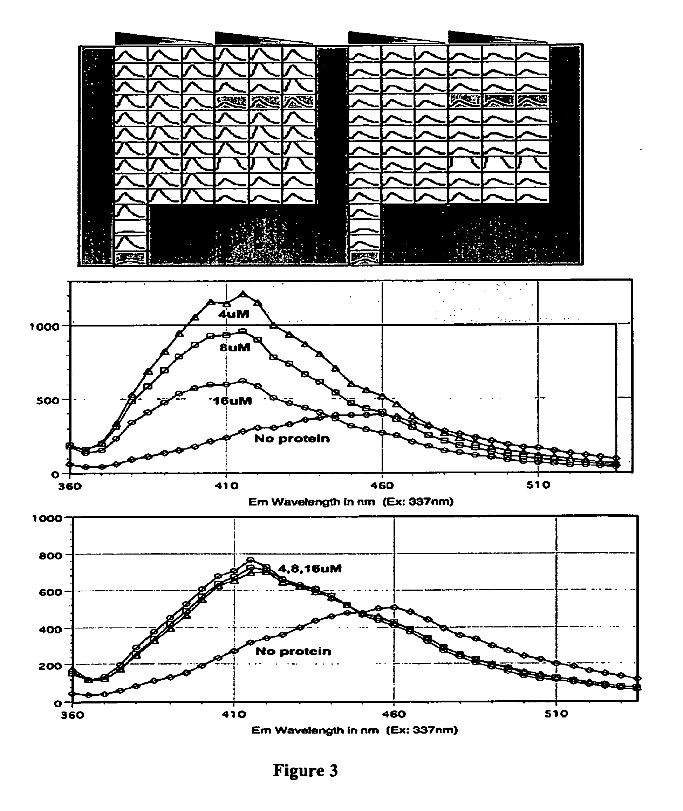

**Figure 3****. An Example of a "Fingerprint" of binding to an chemosensory array.** Image of the plate assay for two different insect OBPs tested for binding to 20 potential ligands. Three concentrations of each ligand was tested: 16uM, 8uM and 4uM. The triangles show the relative concentrations. The control wells are boxed in red. Indole was found to bind one OBP but not the other. These lanes (highlighted in blue) are shown in more detail underneath the plate image. These results demonstrate the feasibility of identifying a VOC by binding to specific insect OBPs.

### DETAILED DESCRIPTION OF THE INVENTION

### Definitions

Unless otherwise stated, all scientific and technical terms used herein have the same meaning as commonly understood by one of ordinary skill in the art to which this invention belongs. Generally, the nomenclature used herein and the laboratory procedures in molecular biology, molecular genetics, biochemistry, physical chemistry, cell culture, protein chemistry, and nucleic acid chemistry described below are those well known and commonly employed in the art. Standard techniques are used for recombinant nucleic acid methods, eukaryotic transformation, and microbial culture and transformation. Enzymatic reactions and purification steps are performed according to the manufacturer's instructions unless otherwise noted. Techniques and procedures are generally performed according to conventional methods in the art. General references include Sambrook et al., Molecular Cloning: A Laboratory Manual, 2nd Ed. (1989) Cold Spring Harbor Laboratory Press, Cold Spring Harbor, N.Y., USA, and Ashburner, M., Drosophila: A Laboratory Manual (1989) Cold Spring Harbor Laboratory Press, Cold Spring Harbor, N.Y., USA. The laboratory procedures described in combinatorial chemistry, synthetic chemistry, and electrophysiology, and the nomenclature used are those well known and commonly employed in the art. As employed throughout the disclosure, the following terms, unless otherwise indicated, shall be understood to have the following meanings

"Allomone" refers to a chemical signal sent from a member of one species to a member of another, with the intent to alter the latter's behavior in favor of the former. Allomones are commonly produced by plant species to affect insect species' behavior.

"Analyte" refers to a substance, molecule, compound, chemical constituent, or other form of matter that is undergoing analysis or detection. An analyte may consist of a single compound or a mixture of compounds.

"Bioinformatics" refers to the discipline that integrates biotechnology and modem computational, statistical, and analytical or mathematical methods.

"cDNA" refers to complementary DNA, which is a DNA copy of the mRNA or messenger RNA expressed in the cell. The term "cDNA" therefore represents gene products or transcripts.

"Chemosensome" refers to the collection of proteins and effectors that enable an insect species to detect chemosensory cues such as scents and/or tastes.

"Chemosensory protein" refers to a protein component of the chemosensory system including the olfactory and gustatory system. Chemosensory proteins can be soluble, insoluble, membrane-bound, extracellular, secreted, or intracellular.

"Domain" refers to an area of a protein with a specific function or exhibiting a specific structural motif.

"Effectors" refers to naturally occurring or synthetic molecules, or compounds capable of interacting with a chemosensory protein under study. Effectors can be agonists, antagonists, or neither.

"Genomics" refers to the cloning and molecular characterization of entire genomes.

"Genetically Modified Organism (GMO)" refers to an organism that has been genetically engineered using gene splicing or molecular biology techniques (vs. a traditional breeding approach) to exhibit specific genetic traits.

"G-protein coupled receptors (GPCRs)" refers to pheromone or odorant serpentine receptors that bind trimeric G-proteins within the cell. Also called odorant receptors (ORs).

"Gustatory receptor" or "GR" refers to the subcellular structures located in the plasma membrane of insect neuronal cells that are responsible for initiating the organism's perception of a specific taste - that is, they allow the organism to taste various flavors or tastes. Also called a GPCR.

'Homologs" refers to genes that have a common ancestry. Homologs are divided into orthologs, which are homologs with the same function as the ancestral gene, and paralogs, that are homologs with a different function from the ancestral gene.

"Homology" refers to the extent of similarity between the DNA sequences encoding two or more genes, or the amino acid sequences comprising two or more proteins, as in a gene or protein family.

"Hydrophobicity" refers to the solubility of a particular protein or molecule in water.

"IPM" refers to integrated pest management, a modem approach to controlling undesirable species by combining natural enemy species such as predators and parasites with other natural control methods and balanced chemical insecticide use.

"Mating disruption" refers to a method of pest control most commonly found in agriculture; it involves saturating the crop environment with a sex pheromone in order to confuse the males and prevent them from locating females.

"Odorant Binding Proteins" or "OBPs" refers to proteins in sensory tissues believed to bind odors, that are typically hydrophobic, and escort them across the hydrophilic extracellular matrix to the cell surface, where odorant receptors are located.

"Odorant Degrading Enzymes" or "ODEs" refers to enzymes that degrade semiochemicals, odors, scents, tastes, or other chemical stimuli in order to re-potentiate the invertebrate chemosensory system.

"Odorant Receptor" or OR refers to the subcellular structures located in the plasma membrane of insect neuronal cells that are responsible for initiating the organism's perception of a specific odor - that is, they allow the organism to smell various scents and odors. Also called a GPCR.

"Odorant" refers to smell, scent, or odor.

"Ortholog" refers to a gene or gene product sharing sequence elements and function with a related gene from another organism. See also "homolog" above.

"PCR" refers to the Polymerase Chain Reaction, a method of amplifying nucleic acid sequences *in vitro* in order to obtain larger amounts of DNA.

"Pheromone" refers to an odorant chemical released by an insect that causes a specific interaction with another insect of the same species.

"Reagents" refers to chemicals and compounds (either naturally occurring or synthetic) or enzymes used in a chemical reaction to measure or yield other substances.

"Reporter Gene" refers to a gene used in biological or biochemical experiments in order to monitor an interaction. Reported genes respond to protein-protein interactions by triggering an effect that is easily detectable, e.g., the emission of fluorescent light or the production of an assayable product.

"RFID" refers to Radio Frequency Identification, a radio-frequency-based, remote data collection technology that uses electronic tags to store data. The tags and the data they contain can be administered (altered) and observed remotely.

"Semiochemicals" refers to chemicals (scents, odors, tastes, pheromones, pheromone-like compounds, or other chemosensory compounds) that mediate interactions between organisms.

"Sensory appendage proteins" or SAPs refers to soluble proteins in sensory tissues believed to bind odors, which are typically hydrophobic, and escort them across the hydrophilic extracellular matrix to the cell surface, where odorant receptors are located.

"Serpentine receptors" refers to GPCRs including GRs or ORs; this term is based on the actual structure of the protein in the cell membrane (seven transmembrane passes in a serpentine shape).

"Signal transduction cascade" refers to a series of molecules in a cell that transduces or converts an external signal (e.g. a pheromone) into a downstream response within the cell (e.g. a change in gene activity).

"Takeout-Like" or "TOL" refers to orthologs of protein encoded by the *Drosophila melanogaster* gene, *takeout.* These proteins may be involved in the regulation of circadian rhythms, in mating, and in feeding behaviors.

"Trans-gene" refers to a gene that has been introduced into the genome of a cell or organism by transformation.

"Transmembrane Domains" refers to hydrophobic domains of a protein that penetrate the cell membrane.

### Introduction

The present invention recognizes the need to construct novel biosensors, novel filtration devices, and novel purification devices, and provides the methods and means to do so by utilizing insect or invertebrate chemosensory proteins in arrays of multiple proteins, smaller arrays or groups of several proteins, or individually. Since insect chemosensory proteins are known to bind a wide array of ligands, the invention describes for the use of numerous proteins sourced from many different insect species in order to maximize the total number of ligands or analytes an array of these proteins is capable of recognizing. The invention also recognizes that despite the tremendous diversity of ligand binding possibilities afforded by insect chemosensory protein arrays, some analytes may not be recognizable by these arrays; furthermore, the characteristics of binding between chemosensory proteins and certain analytes may need to be manipulated. Thus, the invention provides the means and methods necessary to construct chemosensory proteins *in vitro* that feature novel binding characteristics or the ability to bind novel analytes, or to bind known analytes in novel ways with respect to the affinity and/or the kinetics of the interaction. The invention provides distinct advantages over existing methods to identify, concentrate, or purify analytes, since it takes advantage of the diverse ligand binding capabilities and high sensitivity of insect chemosensory proteins.

What follows is a non-limiting introduction to the breadth of the invention, including several general and useful aspects:
1) The invention describes a method to assemble large collections of chemosensory proteins from a variety of insect species into a chemosensory array. The proteins from different species can be identified using bioinformatic analysis and sequence data comparison with known examples that are conserved across species boundaries, or can be discovered by constructing cDNA libraries from sensory tissues of the various species, and expressing the clones *in vitro.* Once available, the chemosensory proteins are assembled into a protein array that has numerous uses in the construction of biosensors, filtration devices, and purification devices. For example, the array or protein components from the array can be incorporated into devices to selectively remove a product or by-product of chemical synthesis, to concentrate a desirable product in chemical synthesis, or to detect a desirable or undesirable analyte in the atmosphere or a solution or liquid.
2) The invention describes a method to construct biosensors based on chemosensory proteins derived from the chemosensory array, or chemosensory proteins capable of binding to a specific analyte or analytes. The chemosensory protein(s) are linked to the reporter system and interaction between them and the analyte(s) can be noted. The sensor apparatus is thus capable of detecting the presence of that analyte.
3) The invention describes the methods and means necessary to use fragments, peptide derivatives, or *de novo* synthesized portions of chemosensory proteins in unique or novel combinations in order to alter the characteristics of binding between the protein and the analyte or ligand. These methods extend the functionality of chemosensory proteins by modifying their binding domains to the point of allowing these domains, and thus the proteins, to bind a given ligand with greater or less affinity or avidity, or of binding altogether novel ligands.
4) The invention provides a method to use a chemosensory protein array in order to generate a chemosensory profile or "fingerprint" for an unknown analyte(s). The protein array is exposed to the unknown analyte, and a reporter system linked to the array is used to note which chemosensory proteins in the array are capable of binding the unknown analyte. This pattern is characteristic of that analyte, and constitutes that analyte's chemosensory profile. Thus, an array can be used to construct a biosensor capable of detecting any analyte the array has been exposed to in the past, regardless of whether the identify or chemical composition of the analyte are known. The invention can be used in a similar manner to generate a chemosensory profile for a known analyte; in either case, a biosensor device can be constructed to detect the presence of the analyte.
5) The invention describes the methods and means necessary to utilize the high selectivity of insect chemosensory proteins in the construction of efficient filtration or purification devices for industrial and chemical uses. For example, the invention recognizes the need to isolate compounds that are chemically identical but arranged in a spatially distinct manner, such as stereoisomers of the same chemical or molecular formula, and provides the means and constructs necessary to do so by utilizing insect chemosensory proteins. Also, the invention recognizes the need to remove pollutants or other undesirable compounds from liquids such as water (*e.g*., for water purification purposes), and provides the means and constructs necessary to do so by utilizing insect chemosensory proteins.

### I. METHODS TO GENERATE A CHEMOSENSORY ARRAY COMPOSED OF CHEMOSENSORY PROTEINS FROM A VARIETY OF INVERTEBRATE SPECIES

Insects use a variety of specialized chemosensory organs or tissues, in which chemosensory proteins are expressed and function. These tissues include the antennae, bristles and other sensory organs throughout the body (including appendages such as the legs or wings), and structures in the head such as the labial and maxillary palps.^{23,26} Since chemosensory proteins are enriched in these tissues, dissecting the tissues and using them to generate cDNA libraries will result in libraries that are enriched for genes expressing chemosensory proteins. Furthermore, gender-specific libraries can be generated from the chemosensory tissues of a particular species, allowing for the rapid identification of gender-specific chemosensory genes such as those genes involved in the mating response. These approaches can be used to rapidly isolate sequences encoding chemosensory proteins from a particular species.^{23,26,27} Chemosensory proteins also can be identified using a variety of other means. One method relies on bioinformatic analysis. For example, OBPs and SAPs have characteristic domains, motifs, and sequence features that are conserved across species boundaries.^{23,26,28} Thus, novel members of these protein families can be identified on the basis of these characteristics by using bioinformatics to compare the sequences of known members expressed in one species to sequences from another species.^{23,26}

Combined, the above approaches can rapidly identify and isolate a wide variety of chemosensory genes from any number of insect species. Given the large number of insect species extant,²⁹ the number of potential chemosensory genes can exceed thousands. These genes can be expressed *in vitro* using a variety of methods common in the art ³⁰⁻³⁴ to generate large collections of chemosensory proteins capable of interacting with a wide variety of analytes. These collections are *chemosensory arrays* and have a variety of applications. The present invention recognizes the need for such chemosensory arrays, and provides means and procedures necessary to incorporate these arrays in devices or biosensors capable of distinguishing or detecting known or unknown analytes or mixtures of analytes; the array can be combined with any number of reporter mechanisms common in the art (enzymatic, electronic, optical, *etc.*) depending on the goals of the specific application.

### II. METHODS TO USE A CHEMOSENSORY ARRAY COMPRISING A VARIETY OF INVERTEBRATE CHEMOSENSORY PROTEINS IN ORDER TO GENERATE CHEMOSENSORY PROFILES FOR UNKNOWN STIMULI

As described above, a chemosensory array can be constructed by expressing a large number of chemosensory proteins expressed *in vitro* using methods common in the art.³⁰⁻³⁴ When coupled to a reporter system (enzymatic, fluorescent, optical, electrical, and so on) such an array is potentially capable of recognizing a wide variety of analytes or chemosensory stimuli. The specific response generated by the array in the presence of a particular analyte is the *chemosensory profile* for that particular analyte; for example, a pheromone such as codlemone³⁵ would be recognized by an array containing chemosensory proteins derived from *Cydia pomonella,* the codling moth, since this organism recognizes the pheromone *in vivo.* In addition to the chemosensory proteins from the codling moth in the chemosensory array, chemosensory proteins derived from other species may also recognize codlemone. By observing the response pattern (yes/no binding for each protein in the array) generated by the reporter system employed in the array, a chemosensory profile specific for codlemone can be generated. This profile is characteristic of the presence of codlemone in any given analyte, and can be used to identify codlemone as a result. (Figure 3)

In order to identify which protein(s) in the chemosensory protein array can bind to a given substance or molecule, several methods can be used. For example, a dye, such as N-phenyl-1-naphthylamine (1-NPN)^{36,37} or (+/-)-12-(9-anthroyloxy)stearic acid (ASA)³⁴ will fluoresce when captured by the ligand-binding pocket of a chemosensory protein and this fluorescence is quenched when the dye is displaced by another molecule. Therefore, spectroscopy or flow cytometry can be used to detect fluorescence quenching and thus identify which chemosensory protein(s) in the array interact with the chemicals of interest.

The same method can be used to identify unknown analytes by generating a characteristic chemosensory profile for them. The array is exposed to the unknown analyte, and the resulting profile recorded for future comparison with other samples potentially containing that same unknown analyte. If the analyte is present, the characteristic chemosensory profile will be generated by the array. This method does not require knowing the identity of the analyte detected.

### III. METHODS TO DEVELOP DEVICES CAPABLE OF DETECTING ENVIRONMENTAL COMPOUNDS EITHER IN THE ATMOSPHERE OR IN AQUEOUS SOLUTION

As described previously in this document, a chemosensory array can be constructed by expressing a large number of chemosensory proteins expressed *in vitro* using methods common in the art.³⁰⁻³⁴ When coupled to a reporter system (enzymatic, fluorescent, optical, electrical, and so on) such an array is potentially capable of recognizing a wide variety of analytes or chemosensory stimuli. The specific response generated by the array in the presence of a particular analyte is the *chemosensory profile* for that particular analyte. The proteins involved in the chemosensory profile for any given analyte can be incorporated into a detection device that relies on chemosensory proteins for analyte "recognition" and is coupled to a reporter mechanism (electrical, mechanical, enzymatic, or other). In this manner, the invention provides the means necessary to construct a biosensor to detect a given analyte in the atmosphere or in an aqueous solution.

### IV. METHODS TO INCREASE THE DIVERSITY OF CHEMOSENSORY PROTEIN RECOGNITION BY UTILIZING PEPTIDES OR DERIVATIVES IN ORDER TO CONSTRUCT HYBRID CHEMOSENSORY PROTEINS IN VITRO AND EMPLOY THESE HYBRID PROTEINS IN A CHEMOSENSORY ARRAY

Chemosensory proteins and their potential ligands have been studied extensively, and in several cases structural studies have been performed or crystal structures have been determined.^{31-33,38-43} To date, chemosensory proteins such as OBPs have been associated with a specific binding partner⁴⁴ or a range of potential binding partners that share structural motifs³⁷ and the specificity of the chemosensory protein:ligand association is conferred by the ligand binding pocket on the chemosensory protein.^{32,38,45,46} Manipulating the binding pocket:ligand interaction can confer novel functionality to the chemosensory protein; this approach has potential uses in biosensors, purification devices, and signal transduction cascades.^{47,48} OBP structure is characterized by conserved domains separated by variable regions, and this overall structure is maintained across species boundaries.⁴¹ Despite the potentially enormous diversity afforded by the large number of chemosensory proteins from the millions of insect species²⁹ there are potentially analytes that are not recognized by any available chemosensory proteins. Furthermore, it may be desirable to manipulate the specificity, strength of ligand binding, or other characteristics of ligand binding between chemosensory protein(s) and an analyte. The present invention provides the means to do so by utilizing peptides or derivatives of chemosensory proteins in order to construct novel, hybrid chemosensory proteins *in vitro.*

DNA sequences encoding fragments of known chemosensory proteins can be cloned from sensory-tissue-specific cDNA libraries^{23,26} or synthesized chemically using a variety of methods, including PCR-based DNA synthesis.⁴⁹ By utilizing linker sequences at the end of each fragment, longer DNA fragments can be assembled into novel sequences encoding proteins with motifs or domains from a variety of sources; this novel motif combination can include complex structures.⁴⁹ The present invention utilizes these methods to generate chemosensory proteins with novel ligand binding domains; these hybrid chemosensory proteins can then be used in chemosensory arrays to recognize novel analytes (Figure 2). The novel binding domains may recognize new ligands, or have altered characteristics with respect to their binding of a given ligand; for example, they would bind a given ligand with an increased or decreased affinity than the wild-type (unaltered) binding domain.

### V. METHODS FOR DEVELOPING DEVICES TO ISOLATE OR DETECT BY-PRODUCTS OF CHEMICAL SYNTHESIS, OR TO ISOLATE OR DETECT DESIRED PRODUCTSOF CHEMICAL SYNTHESIS; ALSO, METHODS FOR DEVELOPING DEVICES FOR PURIFICATION OR CONCENTRATION OF AN ANALYTE FROM A MIXTURE OR SOLUTION

Invertebrate chemosensory proteins are very diverse and are capable of detecting millions of scents, odors, tastes, semiochemicals, or molecules based on their ability to interact with these targets.²³ Chemosensory proteins can be identified and expressed *in vitro* using methods common in the art;^{23,26-28} the expression of numerous such proteins results in a chemosensory protein array as described above. This array can be used to examine a given substance - an odor, scent, or chemical, for example - and identify which available insect chemosensory proteins are capable of binding the substance in question.

Thus, chemosensory proteins can be used in the construction of selective filtering devices that are capable of distinguishing a specific component or intermediate compound in biochemical or industrial processes. Depending on the desired level of selectivity and specificity, a filtering device can be designed to identify classes of related chemicals or it can be designed to be much more selective, to the point of distinguishing stereoisomers of molecules that have identical chemical structures. Or, chemosensory proteins capable of selectively binding to a specific compounds such as a water pollutant compound can be expressed in bacterial cells *(e.g., E. coli* cells) and linked to the bacterial cell membranes such that the chemosensory proteins are present on the outer surface of the bacterial cell membrane. These bacteria can then be used in applications requiring the selective removal of a pollutant compound from water, such as in water purification facilities. The high level of selectivity of insect chemosensory proteins toward analytes has been documented in the Gypsy Moth, *Lemantria dispar*;⁴⁴ this moth's chemosensory system can reliably distinguish stereoisomers of a pheromone.

### VI. METHODS TO DEVELOP PORTABLE DEVICES CAPABLE OF DETECTING EXPLOSIVE SUBSTANCES

The invention recognizes the need to develop devices capable of detecting specific analytes, present in small quantities in the atmosphere or in solution, that are associated with explosives. To accomplish this, a chemosensory array is used to generate a chemosensory profile for each analyte or for commonly used combinations of analytes as described previously, and the portions of the chemosensory array (the chemosensory proteins) that participate in this chemosensory profile are incorporated into a detecting device while coupled to a reporter mechanism.

Analytes of particular interest to this application of the invention include but are not limited to ammonium nitrate (NH₄NO₃); black powder (a mixture of charcoal, sulfur, and saltpeter - nitrates of potassium or sodium); smokeless powder (primarily composed of nitrocellulose); RDX (cyclotrimethylenetrinitramine; also known as cyclonite); C4 (Composition C-4, comprising the explosive, typically RDX or cyclonite; the plasticizer, di(2-ethylhexyl) or dioctyl sebacate; the plastic binder, polyisobutylene; and a tag or marker such as 2,3-dimethyl-2,3-dinitrobutane or DMDNB); other nitroamines such as HMX (cyclotetramethylene-tetranitramine); PETN (Penthrite or Pentaerythritol Tetranitrat); dynamite; TNT (CH₃C₆H₂(NO₂)₃ or trinitrotoluene; a common substance present in military-grade TNT is 2,4-DNT or 2,4-dinitrotoluene); Semtex (a mixture of PETN, RDX, N-phenyl-2-naphthylamine as an antioxidant, a plasticizer such as di-n-octyl phthalate, and a styrene-butadiene rubber binder; later stocks have an ethylene glycol dinitrate tag); TATP (acetone peroxide or triacetone triperoxide); and others.

Insects are known to recognize several of these substances from behavioral assays and from attempts to train honeybees to detect landmines and/or unexploded ordinance (UXO), and for general environmental monitoring.⁵⁰⁻⁵² The present invention provides the methods to develop detection devices based on the chemosensory proteins that enable honeybees and other insects to detect explosives; the invention is novel in that it does not require live insects and merely relies on *in vitro* expression of recombinant chemosensory proteins as described previously in this document. The chemosensory proteins from honeybees and other insects capable of detecting analytes of interest are used to construct an explosives-specific chemosensory array that is coupled to an enzymatic, electric, mechanical, or other form of reporter mechanism to comprise a biosensor for detecting explosives.

The invention can also be used to generate a detector mechanism specifically designed to detect a known marker compound that is incorporated into explosive formulations by manufacturers. This arrangement is intended to aid in the rapid identification of the source of a tested explosive sample.

### VII. METHODS FOR DEVELOPING A BIOSENSOR WITH INDUSTRIAL FOOD PREPARATION APPLICATIONS

The invention recognizes the need to detect byproducts of food decomposition in order to ensure safe food processing in applications such as meat packaging and other food preparation applications. Thus, the invention provides the methods necessary to identify analytes commonly associated with food decay or food quality problems. To accomplish this, a chemosensory array is used to generate a chemosensory profile for each analyte or for commonly occurring combinations of analytes as described previously in this document, and the portions of the chemosensory array (the chemosensory proteins) that participate in this chemosensory profile are incorporated into a detecting device while coupled to a reporter mechanism. These devices can be used in packaging plants, other industrial food preparation plants, food preparation applications (restaurants, mess halls, *etc*.), or, in a portable format, by health inspectors or other food service professionals.

This aspect of the invention can be incorporated directly into packaging materials and coupled to a reporter mechanism such as an enzymatically triggered color change or other mechanism, to allow end users or consumers to quickly determine if a particular food article is spoiled. If this aspect of the invention is coupled to a Radio Frequency Identification (RFID) mechanism, it will enable shipments of food or other perishable items to be monitored remotely for spoilage.

Analytes of particular interest to this application of the invention include but are not limited to fermentation products such as alcohol(s) produced by microorganisms, byproducts such as lactic acid, and ptomaines (nitrogenous organics resulting from bacterial putrefaction of protein) including putrescine, cadaverine, parvolin, and sepsin. The invention also provides the methods necessary to detect common invertebrate infestations in food products, again using a chemosensory array profile approach to determine which chemosensory proteins can detect compounds for a particular species.

The devices described in this method could also include functionality from the following method, "Methods to develop a portable device capable of detecting the presence of insect pests" in order to extent their application to the detection of pest-infested fruits and vegetables. This application is of interest to food packers, food exporters, agricultural inspectors, and customs agencies.

The invention also recognizes the need to monitor food quality to ensure product consistency while producing foodstuffs such as coffee and wine, where the aroma of the final product is critical to commercial success and trained humans capable of detecting specific odors or odor combinations have traditionally been charged with the task of monitoring the quality of production based on aroma. The method described here can also be used in this instance; the invention provides the means necessary to identify the key volatile components of a coffee or wine that contribute toward a desirable aroma by using a chemosensory protein array to develop a scent fingerprint for that coffee or wine. Production quality is monitored by checking samples of the coffee or wine using a biosensor with insect chemosensory proteins that recognize the volatiles emitted by a control or reference coffee or wine sample to ensure the aroma emitted by tested samples corresponds to the fingerprint of the reference.

### VIII. METHODS TO DEVELOP A PORTABLE DEVICE CAPABLE OF DETECTING HUMAN CORPSES WITHIN RUBBLE OR IN DISASTER AREAS; ALSO, METHODS TO DEVELOP A PORTABLE DEVICE CAPABLE OF DETECTING SURVIVING HUMANS WITHIN RUBBLE OR IN DISASTER AREAS

The invention recognizes the need to detect byproducts of corpse decomposition in order to locate human remains. Thus, the invention provides the methods necessary to identify analytes commonly associated with tissue decay or tissue decomposition. To accomplish this, a chemosensory array is used to generate a chemosensory profile for each analyte or for commonly occurring combinations of analytes as described previously in this document, and the portions of the chemosensory array (the chemosensory proteins) that participate in this chemosensory profile are incorporated into a detecting device while coupled to a reporter mechanism. These devices can be used in portable detection mechanisms that can be transported to the site of an accident, attack, bombing, natural disaster, or other incident with multiple casualties, and used in *lieu* of or in addition to canine search units.

Analytes of particular interest to this application of the invention include but are not limited to fermentation products such as alcohol(s) produced by microorganisms, byproducts such as lactic acid, and ptomaines (nitrogenous organics resulting from bacterial putrefaction of protein) including putrescine, cadaverine, parvolin, and sepsin.

Similar methodology can be used to develop a chemosensory profile for living humans; analytes that are of interest in this application are the volatiles in the breath and those emitted from the skin of living humans.⁵³ This chemosensory array can be coupled to a reporter mechanism in order to generate a device to detect survivors in disaster areas.

### IX. METHODS TO DEVELOP A PORTABLE DEVICE CAPABLE OF DETECTING THE PRESENCE OF INSECT PESTS

Many insect species employ chemical communication and emit scents or odors in order to communicate with other individuals of their species, or as signals that can be recognized by other species for a variety of reasons.^{29,37,53-57} Plants also interact with insects using chemical signaling; plant species are known to emit allomones or other chemical signals to attract beneficial insects.⁵⁸ These chemical signals regulate critical aspects of insect behavior, such as feeding, foraging; oviposition, or mating.²³ Due to the importance of chemical communication, insects have developed highly sensitive chemosensory systems; insect chemosensory proteins are thus ideally suited to recognize these chemical signals.

The present invention provides the means necessary to develop a detector device capable of identifying a particular species of insect based on detecting the chemical signals characteristic of that particular insect species. The applications of this aspect of the invention are mainly in pest control, whether agricultural, domestic, Homeland Security, or public health related. To accomplish this, a chemosensory array is used to generate a chemosensory profile for each analyte or for commonly occurring combinations of analytes as described previously, and the portions of the chemosensory array (the chemosensory proteins) that participate in this chemosensory profile are incorporated into a detecting device coupled to a reporter mechanism. The chemosensory array may be presented with a pheromone or other semiochemicals characteristic of a species of interest, or simply with extracts from ground specimens of the species of interest. This aspect of the invention can also be coupled to a Radio Frequency Identification (RFID) mechanism to allow remote monitoring of infestations.

One group of insects that potentially represent a promising source of chemosensory proteins is parasitoids such as parasitic wasps. These insects specialize in locating host organisms such as moths, and are thus often used in integrated pest management (IPM) schemes as a means of controlling insect pest populations.^{59,60} The invention provides for isolating chemosensory proteins from parasitoid wasp species, and other species that specialize in the chemical identification of pests; the chemosensory proteins can be expressed *in vitro* and used to assemble a chemosensory array capable of detecting the pest species in question when coupled to a reporter mechanism. Such a construct can be incorporated into a detection device to be used commercially.

### X. METHODS TO DEVELOP A DEVICE CAPABLE OF DETECTING DISEASE IN HUMANS

Sensors to detect disease in humans have been the topic of numerous investigations, with recent emphasis on the development of the "electronic nose".^{5,10} The presence of specific volatiles has been associated with numerous diseases in humans. For example, cancerous cells on the skin or inside the body emit volatile compounds into the blood, the atmosphere or body fluids that are then expelled; the volatiles can be detected in order to speed the identification or diagnosis of disease. This is the case with a diverse set of human diseases such as prostate cancer,⁶¹⁻⁶³ bladder cancer,⁶⁴ pulmonary tuberculosis,⁶⁵ breast cancer,⁶ lung cancer,^{66,67} melanoma,⁶⁸ angina,⁶⁷ and diabetes.^{9,69-71} In addition, anecdotal evidence suggests the emission of volatiles from the human body that are detectable by animal chemosensory systems in the case of imminent epileptic seizures; epileptic owners of service dogs often report their canines have developed the ability to sense an imminent seizure event before the patients can detect physiological changes themselves.
The invention recognizes the need to enhance the sensitivity and versatility of electronic nose-type biosensor devices, and provides the means necessary to do so. To accomplish this, the volatiles associated with a particular disease in humans are introduced as analytes into the insect chemosensory protein array described elsewhere in this document. The chemosensory array is used to generate a chemosensory profile for each analyte or for commonly occurring combinations of analytes as described previously in this document. As a control, the volatiles associated with healthy humans are used; the chemosensory fingerprint or profile generated by the analytes emitted by healthy humans is compared to that from diseased humans in order to identify those analytes positively associated with a particular disease. The portions of the chemosensory array (the chemosensory proteins) that participate in the chemosensory profile of a disease are incorporated into a detecting device while coupled to a reporter mechanism. These devices can be used in clinics, hospitals, portable health care labs, field hospitals, humanitarian relief efforts, and other health care or diagnostic applications.

### XI. METHODS TO DEVELOP A DEVICE CAPABLE OF DETECTING VOLATILES SUCH AS FUMES

Some insect species rely on their ability to detect the byproducts of combustion. For example, jewel beetles (*Melanophila spp*.) must locate freshly burned forest areas in order to oviposit, as the larvae of this species can only develop in the wood of freshly burned trees.⁷² Thus, these insects may be able to locate burned or burning wood from distances of several km by detecting volatiles such as phenolic compounds typical of burnt forest areas; one example of such a compound is guaiacol⁷² (C₇H₈O₂).

The invention provides the means to generate a chemosensory array comprising chemosensory proteins from numerous insect species, including species with a known sensitivity to phenolic compounds and/or combustion byproducts. This array can be used to isolate the specific chemosensory proteins responsible for insect sensitivity to burning wood, and these chemosensory proteins can be used in novel fire detection devices when coupled with an electric or electronic reporter system or alarm. The invention thus provides the means to replace or augment existing smoke detectors (based usually on the radioactive compound, americium oxide, AmO₂). Other applications of the invention include forest fire detection systems, and commercial or industrial fire detection systems for use in warehouses, airports, schools, auditoria, sporting venues, public transport stations, convention centers, public gathering places, *etc.*

### XII. METHODS TO DEVELOP COMPOUNDS OR CONSTRUCTS THAT MASK ODORS, SCENTS, OR OTHER SEMIOCHEMICALS

The present invention recognizes the need to develop compounds, devices, or constructs capable of masking semiochemicals in industrial, agricultural, and domestic environments. The invention therefore describes methods and means to incorporate chemosensory protein-based odor-masking technologies into a variety of commercial products or devices. Chemosensory proteins that can be used include OBPs, odorant degrading enzymes, sensory appendage proteins, or chemosensory receptors.

DNA sequences encoding OBPs or other chemosensory proteins can be expressed *in vitro* using tools common in the art. Devices incorporating these chemosensory proteins in a solid form, particulate form in suspension, or aqueous form in a gel have numerous commercial, agricultural, and domestic applications that include but are not limited to the following:
a. Deodorizing products deployed in public or domestic lavatories found anywhere from airports to homes.
b. Deodorizing products used in the home, inside refuse containers. Furthermore, chemosensory protein-based compounds be incorporated into the composition of refuse containers themselves to generate odor-resistant containers.
c. Particulate or liquid products based on chemosensory proteins can be introduced in commercially available natural fertilizer to mask the fertilizer's odor in applications requiring an odor-free environment.
d. Products be incorporated into domestic and industrial cleanser products, and deployed in diverse environments ranging from school cafeterias to slaughterhouses, food processing plants, and restaurants.

Products incorporating chemosensory proteins can mask the unpleasant odors associated with cigarette, cigar, and pipe smoke. The products can be introduced as an aerosol spray or can be incorporated directly into cigarettes, cigars, or smoking tobacco.

These methods can also be employed to target chemosensory proteins including sensory appendage proteins (SAPs), odorant binding proteins (OBPs), odorant degrading enzymes (ODEs), and other proteins involved in olfaction, gustation, chemosensation, or the regulation of chemosensory-mediated behavior.

Furthermore, products to mask or trap odors need not incorporate the entire chemosensory protein; they can instead incorporate peptide derivatives or fragments.

### EXAMPLES

### EXAMPLE 1: GENERATING A CHEMOSENSORY ARRAY COMPOSED OF CHEMOSENSORY PROTEINS FROM A VARIETY OF INVERTEBRATE SPECIES.

Many important aspects of insect behavior rely on chemosensory cues.^{23,73,74} Since chemosensation profoundly influences insect behavior, the molecules and processes involved in the chemosensory pathway have developed extreme sensitivity toward chemosensory stimuli or analytes. Insects are capable of recognizing a tremendously diverse number of analytes at very low concentrations. The molecular mechanism of analyte detection in insects has been extensively studied in various species.^{23,26,27,75-89} The chemosensory signal transduction cascade is facilitated by extracellular, transmembrane, and intracellular proteins.⁹⁰⁻⁹² The major molecular participants are odorant binding proteins (OBPs), G-protein coupled receptors (GPCRs), sensory appendage proteins (SAPs), odorant degrading enzymes (ODEs), circadian rhythm proteins (such as TOLs), gustatory binding proteins, and gustatory receptors (GRs).

These proteins can be expressed *in vitro* using methods common in the art.^{23,26,27} Numerous methods are available to identify and isolate chemosensory proteins from a wide array of species, including bioinformatic analysis to search for orthologs of known proteins across species boundaries, and the construction of chemosensory-specific cDNA libraries from the species of interest from which clones encoding chemosensory proteins can be expressed. By combining numerous chemosensory proteins from a number of species, an array of chemosensory proteins can be constructed. Since the array contains chemosensory proteins from numerous species, it is capable of recognizing more analytes than any one insect species alone. The array is linked to a reporter system common in the art, such as a mechanical, electrical, piezoelectric, enzymatic, or electronic system, and can report the presence of an analyte in a mixture of gases or in a liquid solution by exposing each protein in the array to the specimen and noting whether the reporter system registers an interaction (binding event) between any protein in the array and the analyte of interest.

Numerous insect chemosensory proteins have a list of known natural ligands, that is, analytes they interact with *in vivo.*^{31,75,93-96} Arrays can be constructed with constituent proteins that emphasize recognition of analytes for a given purpose; for example, arrays that specialize in the recognition of alcohol analytes can be constructed using known chemosensory proteins that bind alcohols and their orthologs or structurally similar proteins from a wide variety of insect species. Furthermore, the known ligand binding partners serve as positive controls to test array and reporter system functionality.

### EXAMPLE 2: USING A CHEMOSENSORY ARRAY COMPRISING A VARIETY OF INVERTEBRATE CHEMOSENSORY PROTEINS IN ORDER TO GENERATE CHEMOSENSORY PROFILES OR FINGERPRINTS FOR UNKNOWN STIMULI.

As shown in Example 1, a chemosensory array comprising chemosensory proteins from a wide variety of insect species expressed *in vivo* and linked to a reporter system common in the art can be constructed. Such an array is capable of recognizing a wide variety of analytes either in solution or in the atmosphere, and array function can be tested using analytes known to interact with chemosensory proteins present in the array.

The location and identity of each chemosensory protein in the array is known. Since chemosensory proteins individually recognize (bind to) one or a small number of analytes, that is, since chemosensory proteins tend to have specific ligands,²³ the reporter system used will generate a distinct pattern of recognition when the array is presented with an analyte of interest, and this pattern of recognition will be unique to that analyte (Figure 3). Thus, the array can be used to generate *chemosensory profiles* or *chemosensory fingerprints* for tested analytes.

These chemosensory fingerprints have numerous applications. For example, an analyte of interest can be recognized easily if the chemosensory array's reporter system generates a chemosensory fingerprint known to arise when that analyte is present. This is an efficient way of constructing diverse biosensors, and the underlying chemosensory array can be common (mass produced) yet used in devices that detect a wide array of analytes (from natural gas leaks to explosives to spoiled food to the presence of insect pests on the basis of released odors).

Chemosensory fingerprints are also useful in the efficient construction of detector devices that can reveal the presence of an analyte or mixture of analytes without necessarily knowing the source of those analytes. For example, if a novel mycobacterium or other species capable of causing disease in humans by infecting the lungs is introduced into the United States by an infected traveler from the developing world, the bacterial infection generates volatiles in the patient's breath^{5,65} that may be detectable by a chemosensory array like the ones described here. A chemosensory array with a reporter mechanism can be exposed to the patient's breath to generate a chemosensory fingerprint of the volatile present from that particular individual. Comparison to a fingerprint from uninfected individuals will reveal the chemosensory proteins that are responding to the volatiles in the patient's breath that are the product of the unknown pathogen. This chemosensory profile can then be used to design novel biosensors to rapidly diagnose the new disease; these biosensors may use an enzymatic, electric, mechanical, or other reporter system to alert the user to the presence of an infected individual that breaths into a mouthpiece. Knowledge of the identity of the pathogen is not necessary, nor is knowledge of the identify of the specific volatiles in the breath created by the pathogen in the lungs. Such a biosensor can be deployed at airports, ports of entry, health car clinics, hospitals, or other areas where infected individuals may be identified.

### EXAMPLE 3: DEVELOPING A DEVICE TO DETECT ENVIRONMENTAL COMPOUNDS IN THE ATMOSPHERE.

The concept of a chemosensory fingerprint as described in Example 2 has numerous applications, including the construction of a biosensor for detecting the presence of compounds in the environment or atmosphere. For example, a biosensor based on insect chemosensory proteins in an array that is linked to a reporter system such as an audible beeper or alarm can be constructed to detect molecules that are colorless and odorless to humans, thus making these compounds difficult for humans to detect.

Such a detector is particularly useful in the detection of toxic compounds in the atmosphere that pose a health risk to humans, as humans are often unable to sense them using their own chemosensory systems, or the chemicals are so toxic that they must be detected before they are smelt by humans. Examples of such chemicals are the nerve gases, tabun (GA), soman (GD), sarin (GB), cyclosarin (GF) and VX. These agents are used in chemical weapons and are extremely toxic to humans, making their detection important to security, military, and anti-terrorist applications.

In this example, the invention provides the means and methods necessary to detect the nerve gas, soman (1,2,2-Trimethylpropyl methylphosphonofluoridate). Soman is a volatile, colorless liquid classified as a weapon of mass destruction by United Nations Resolution 687. The stockpiling and production of soman has been banned by the Chemical Weapons Convention of 1993. A chemosensory array comprising chemosensory proteins from numerous insect species can be used to identify insect chemosensory proteins capable of detecting soman; these proteins are then incorporated into a specialized array that is linked to a reporter system such as a piezoelectric buzzer with a power source, and incorporated into portable devices. These devices are biosensors capable of detecting soman and can be used in military, homeland security, and government installations. Portable devices can be used in the field by armed forces personnel and weapons treaty compliance inspectors.

### EXAMPLE 4: DEVELOPING DEVICES TO ISOLATE OR DETECT BY-PRODUCTS OF CHEMICAL SYNTHESIS, OR TO ISOLATE OR DETECT DESIRED PRODUCTS OF CHEMICAL SYNTHESIS OR FOR PURIFICAT10N OR CONCENTRATION OF AN ANALYTE FROM A MIXTURE OR SOLUTION.

Insect chemosensory proteins have been shown to selectively bind to a particular stereoisomer of a ligand and not the another stereoisomer of the same ligand; for example, the moth, *Lemantria dispar,* can use its chemosensory system to select a specific stereoisomer of a pheromone.⁴⁴ This extremely selective, highly specific binding activity of chemosensory proteins can be utilized to select for, concentrate, select against, or purify a specific analyte from a complex mixture, and has numerous applications in industry. For example, chemosensory proteins from a wide variety of insect species in an array linked to a reporter system can be exposed to diastereomers of ephedrine, a drug used in a variety of applications and sold over the counter as well as in prescription medications. Each diastereomer will generate a unique chemosensory profile, and those chemosensory proteins capable of binding one diastereomer but not the other(s) can be selected for inclusion into purification devices. These devices are then incorporated into synthesis pathways by pharmaceutical companies in order to efficiently isolate or purify desired products from mixtures containing their stereoisomers and/or by-products of chemical synthesis. Thus, a chemosensory protein with highly selective specificity of binding for the (*1S*,*2S*)-diastereomer of ephedrine, (called "pseudoephedrine" and available over the counter as a decongestant) is used to isolate and purify this stereoismoer from a mixture of compounds including (*1R*,*2S*)-ephedrine (banned for over the counter use in diet aids; used for asthma) and/or other structurally related amines. Likewise, selecting any dextrorotatory isomer from its levorotatory isomer, or *vice versa*, can be accomplished by using a highly; selective chemosensory protein identified from the array and incorporated into a filtering mechanism in a pharmaceutical production line. For example, all molecules containing a characteristic structure such as the 17 carbon atoms arranged in four rings (hallmark of steroid molecules) can be rapidly identified, isolated, or concentrated by using chemosensory proteins capable of selectively binding them.

This aspect of the invention is also useful in applications such as environmental cleanup or any application requiring the selective removal of a class of compounds or a single compound.

### EXAMPLE 5: USING CHEMOSENSORY A CHEMOSENSORY PROTEIN ARRAY TO DEVELOP PORTABLE DEVICES CAPABLE OF DETECTING EXPLOSIVE SUBSTANCES.

Live insects are already in use to detect unexploded ordinance (UXO). Insects are known to recognize several explosive substances, ⁵⁰⁻⁵² and honeybees are being trained to detect land mines or other UXO. However, there are several serious drawbacks to using live insects for detecting UXO. Firstly, although insects have very sensitive chemosensory systems as a group, the variety of compounds any individual species can detect is less than the variety of compounds numerous species can detect. Secondly, insects must be available in large numbers, as most species have a short life span; this implies domesticated species such as the honey bee are easier to use in detection than other species that may have equally sensitive chemosensory systems, such as wasps. Thirdly, insects like honeybees must be trained to detect explosives, and must be observed detecting these explosives in order to identify the location of the UXO.

The present invention overcomes these difficulties and describes the methods to develop detection devices based on the chemosensory proteins to detect explosives without requiring live insects. The chemosensory proteins from honeybees and other insects capable of detecting analytes of interest are used to construct an explosives-specific chemosensory array that is coupled to an enzymatic, electric, mechanical, or other form of reporter mechanism to comprise a biosensor for detecting explosives. For example, a chemosensory array containing chemosensory proteins from several hundred insect species is used to develop a chemosensory profile for the commonly available explosive, Semtex. This substance is a commercially produced mixture of PETN, RDX, N-phenyl-2-naphthylamine, di-n-octyl phthalate, and a styrene-butadiene rubber binder. The array will provide chemosensory proteins capable of binding to each of these substances individually, or to mixtures of the component substances. These chemosensory proteins can be incorporated into a portable device and linked to a piezoelectric buzzer for use as an explosives alarm system in battlefields, airports, or other military or security applications.

### EXAMPLE 6: HIGH-THROUGHPUT SCREENING TO ISOLATE A MODIFIED OBP(S) WITH HIGHER BINDING AFFINITY TO 2,4-DINITROTOLUENE (2,4-DNT) FOR USE IN A BIOSENSOR TO DETECT EXPLOSIVES.

Although insect chemosensory proteins represent a highly diverse group with a wide variety of binding properties there are potential analytes that may be unrecognized by existing expressed proteins. Furthermore, it may be desirable to manipulate the specificity, strength of ligand binding, or other characteristics of ligand binding between chemosensory protein(s) and analyte. This example shows how a modified OBP with a higher binding affinity to a given analyte can be can be isolated so that it can be used in a biosensor device to detect explosives.

The compound 2,4-DNT is a chemical degradation by-product of trinitrotoluene, a common component of many high explosives. Several groups have focused on detection of 2,4-DNT as a method to locate explosives, including underwater mines and land mines. At least one such study successfully used honeybees (*Apis mellifera*) trained to smell 2,4-DNT to locate land mines,^{50,97} indicating insects have chemosensory proteins that recognize this compound. The *Apis* genome has been sequenced and many of the genes encoding chemosensory proteins have been identified.

2,4-DNT would be screened using the chemosensory array described above. A variety of chemosensory proteins from the array would be identified that bind 2,4-DNT with a range of affinities. In order to isolate proteins with improved affinity, DNA sequences representing the individual functional domains of chemosensory proteins that bind 2,4-DNT are used to generate oligomers of approximately 60bp. Since these genes are on average 300bp long this means generating about 5 oligomers from each gene. In addition, linking oligomers of 30bp are generated representing the reverse strand, these contain 15bp from the end of two adjacent 60bp oligomers. Both sets of oligomers are mixed together, allowed to anneal and a DNA polymerase is used to fill in the gap. This method is a novel and substantial improvement of the method detailed by Xiong *et al.* ("A simple, rapid high-fidelity and cost-effective PCR-based two-step DNA synthesis method for long gene sequences").⁴⁹ By mixing oligomers from the 2,4-DNT-binding chemosensory proteins with specific linking oligomers we can create novel gene chimeras containing functional domains from the different proteins. These novel genes are expressed and the resulting proteins screened for improved binding affinity to 2,4-DNT using an assay that has been previously-described above.

The improved protein can then be produced and incorporated into a detecting device while coupled to a reporter mechanism.

### EXAMPLE 7: DEVELOPING A BIOSENSOR TO REMOTELY TRACK FOOD SHIPMENTS AND DETECT FOOD SPOILING OR DEGRADATION.

The food and shipping industries require a reliable method to detect byproducts of food decomposition in order to ensure safe food processing in applications such as meat packaging and other food preparation applications. Meats and vegetables are often prepared in a central location such as a packing plant and shipped across country or even abroad as perishable cargo, and care must be taken that this cargo arrives at the destination still fit for human consumption.

Decomposing meat typically harbors bacteria and other microorganisms that produce characteristic substances such as alcohol(s), lactic acid, and nitrogenous organics resulting from bacterial putrefaction of protein including ptomaines, putrescine, cadaverine, parvolin, and sepsin. The invention can use insect chemosensory proteins to detect these substances. A chemosensory array composed of numerous chemosensory proteins from different insect pest species, particularly species known to be attracted to cadavers and carcasses, is used to generate a chemosensory profile for each analyte or for commonly occurring combinations. Those proteins from the chemosensory array that do recognize the analytes of interest are then incorporated into a detecting device while coupled to a reporter mechanism. Thus, the invention can be incorporated directly into packaging materials and coupled to a reporter mechanism such as an enzymatically triggered color change or other mechanism, to allow end users or consumers to quickly determine if a particular food article is spoiled. A Radio Frequency Identification (RFID) mechanism can also be linked to the biosensor, so as to enable shipments of food or other perishable items to be monitored remotely for spoilage while in transit on ships, trucks, trains, airplanes, or other means of transport. The RFID-linked spoilage biosensor can also monitor foodstuffs in storage in warehouses *etc*.

### EXAMPLE 8: DEVELOPING A BIOSENSOR TO CHECK QUALITY OF FOODS OR BEVERAGES (SUCH AS COFFEE) BASED ON SCENT.

The invention recognizes the need to detect a specific aroma or combination of aromas or scents as an indicator of quality in certain foods or beverages. For example, the coffee and wine industries rely on humans that are trained to detect specific odors or aromas in these products; the quality of coffee or wine is directly associated to its aroma by consumers and producers alike. The invention provides the means necessary to identify the key volatile components of a coffee or wine that contribute toward a desirable aroma; once these volatiles are identified they are incorporated into a chemosensory fingerprint and the interested industry can then monitor coffee production to ensure current batches also emit the same volatile scents or odors in order to obtain a consistent aroma.

A sample of coffee with a highly desirable aroma (a reference coffee) is used to establish the chemosensory fingerprint of the "correct" or desirable aroma for subsequent production. A chemosensory protein array consisting of numerous insect chemosensory proteins linked to a reporter mechanism (see Figure 3) is exposed to the volatiles aromas and scents produced by the reference coffee in order to identify those chemosensory proteins that are capable of binding to the molecules emitted. The recognition pattern generated by the protein array is representative of the reference coffee; it is the reference coffee's chemosensory fingerprint (see Figure 3). Production quality is then be monitored by checking subsequent samples of the coffee ensure the scent emitted by those samples corresponds to the fingerprint. Thus, the invention can be used to replace or supplement trained humans in scent testing coffee, wine, or other products.

### EXAMPLE 9: A BIOSENSOR TO DETECT SURVIVORS TRAPPED IN RUBBLE, MINESHAFTS, OR OTHER AREAS WHERE THEY CANNOT BE SEEN.

The invention recognizes the need to detect byproducts of human respiration, scents or odors such as oils and sweat emitted from the skin, and other odors characteristics of living humans in order to construct a biosensor for the discovery of survivors of natural or wartime disasters that may be trapped under wreckage, rubble, mines, *etc.*

Numerous insect species have chemosensory proteins specialized for the detection of humans; for example, the malaria carrying mosquito, *Anopheles gambiae,* is anthropophilic and can locate humans in the dark by sensing volatiles in the human breath and emitted from the skin.^{23,98-100} The invention recognizes the application of chemosensory proteins from insects that can detect human-emitted volatiles in the construction of biosensor devices to detect humans. Thus, the invention provides the methods necessary to identify analytes commonly associated with the presence of living humans. To accomplish this, a chemosensory array is used to generate a chemosensory profile for each analyte or for commonly occurring combinations of analytes typically remitted by humans, such as carbon dioxide and lactic acid.^{23,98-100} As described previously in this document, the portions of the chemosensory array (the chemosensory proteins) that participate in this chemosensory profile are incorporated into a detecting device while coupled to a reporter mechanism. These devices can be used in portable detection mechanisms that can be transported to the site of an accident, attack, bombing, natural disaster, and used in *lieu* of or in addition to canine search units.

### EXAMPLE 10: A BIOSENSOR TO DETECT THE PRESENCE OF THE CODLING MOTH, CYDIA POMONELLA, AN INSECT PEST SPECIES.

Insects are economically detrimental to many industries, such as the agriculture, food shipment, and housing industries. These industries invest large sums of money to control insect pests, primarily with insecticides and/or other forms of pest management such as IPM. This invention provides the means to construct a biosensor capable of detecting the presence of a particular insect pest species; such a device is useful in the field, in packaging plants, as well as in inspection stations such as those run by the government agriculture departments of many nations to control import/export of infected foods.

Numerous insect species employ chemical communication and emit scents or odors in order to communicate with other individuals of their species, or as signals that can be recognized by other species for a variety of reasons.^{29,37-53-57} Plants also interact with insects using chemical signaling; plant species are known to emit allomones or other chemical signals to attract beneficial insects.⁵⁸ These chemical signals regulate critical aspects of insect behavior, such as feeding, foraging, oviposition, or mating.²³ Due to the importance of chemical communication, insects have developed highly sensitive chemosensory systems; insect chemosensory proteins are thus ideally suited to recognize these chemical signals.

The codling moth, *Cydia pomonella*, is a pest of pome fruit in the United States. The codling moth utilizes a pheromone, codlemone, to allow individuals to locate one another for mating; the structure and function of codlemone are well characterized, and it is known that male moths respond to it.¹⁰¹⁻¹⁰⁴ Currently, levels of codling moth infestation in apple orchards are monitored using traps combining a glue to hold male moths with artificial codlemone to attract them, and require human intervention. This invention provides the means to develop a codling moth biosensor that detects moths by identifying codlemone in the atmosphere. A chemosensory array containing chemosensory proteins from the codling moth is generated by expressing these proteins *in vitro.* The array can contain chemosensory proteins from other insect species, as it is likely several other species express proteins that can identify codlemone. For example, predators such as wasps that feed on codling moth are likely to express chemosensory proteins that can detect volatile scents or odors emitted by the moths; the protein array can thus include chemosensory proteins from wasp species. The array is used to generate a chemosensory profile for codlemone. The chemosensory proteins that participate in this chemosensory profile are incorporated into a detecting device while coupled to a reporter mechanism. This biosensor device can also be coupled to a Radio Frequency Identification (RFID) mechanism to allow remote monitoring of infestations.

Apples infested with codling moths are banned in several countries that import fruit from the United States. Thus, this aspect of the invention coupled to an RFID mechanism can be used to monitor fruit shipments for possible codling moth infestation during transit, ensuring no infested shipments are allowed to arrive.

### EXAMPLE 11: A BIOSENSOR TO DETECT TUBERCULOSIS IN HUMANS.

Several human diseases cause the patient to emit detectable volatiles either through the skin, breath, or body fluids. One such disease is pulmonary tuberculosis,⁶⁵ a contagious disease in humans that is caused by mycobacteria. Tuberculosis (TB) infection among humans in the developed world resurged in the 1990s as a result of immigration, relaxed TB control in countries where the disease had previously been eradicated, and an increase in the number of immunosuppressed patients as a result of AIDS.

The established method to test a human for exposure to the organism that causes TB was developed in the early 20^{th} century and named after French physician, Charles Mantoux. The Mantoux test involves an intradermal injection of 10 Tuberculin units, usually performed on the inner forearm of the test subject. The subject is observed for visible reaction to the Tuberculin at the site of injection up to 72 hours later. One drawback to the Mantoux test is the lag period between injection and analysis. Another drawback is that the test does not confirm active tuberculosis; rather, it merely confirms the subject was at one point exposed to the microorganism. Furthermore, the results must be interpreted, essentially in a subjective manner, depending on the history of the subject. For example, induration (measured in millimeters) of 5 mm is positive in an HIV patient but negative in lab personnel, drug users, or diabetics; these latter persons are considered positive at 10 mm.

The invention describes the means to construct a biosensor for rapid diagnosis of active TB in humans based on detecting the volatiles associated with pulmonary TB in the breath of tested individuals. This device can be used at ports of entry, airports, health clinics, and other areas where high population densities dictate the need for careful public health screening (dorms, military bases, *etc*.). To accomplish this, the volatiles associated with pulmonary TB in humans tested using an insect chemosensory protein array containing insect chemosensory proteins expressed *in vitro* and sourced from numerous insect species. The chemosensory array is used to generate a chemosensory profile for active TB infection in humans as described previously in this document, and the portions of the chemosensory array (the chemosensory proteins) that participate in this chemosensory profile are incorporated into a detecting device while coupled to a reporter mechanism. The breath of non-infected humans is used as a control, to exclude chemosensory proteins that recognize normal analytes in human breath from the array in the TB biosensor. These devices can be used in clinics, hospitals, portable health care labs, field hospitals, humanitarian relief efforts, and other health care or diagnostic applications. The devices can also be coupled to a RFID mechanism to allow rapid data collection by public health agencies.

### EXAMPLE 12: DEVELOPING A BIOSENSOR TO DETECT A FOREST FIRE REMOTELY.

Jewel beetles (*Melanophila spp*.) must locate freshly burned forest areas in order to oviposit; thus, this species relies on its chemosensory system to detect forest fires.⁷² These beetles can detect burning wood from distances of several km by identifying phenolic compounds such as guaiacol⁷² (C₇H₈O₂).

The invention describes the means to construct a biosensor based on insect chemosensory proteins that can be used to detect forest fires remotely and signal the appropriate authorities. A chemosensory array comprising chemosensory proteins from numerous insect species, including species with a known sensitivity to phenolic compounds and/or combustion byproducts, is used to identify the proteins responsive to the smoke generated from tree fires. These chemosensory proteins can be used in novel fire detection devices when coupled with an electric or electronic reporter system or alarm. For example, the array proteins are incorporated into a small device with a power supply and an electric reporter mechanism that activates an RFID transmitter when the array proteins detect smoke. The transmitter can be monitored by fire control agencies remotely. Other applications of this device include airports, warehouses or storage facilities, shipping, ports of entry, fuel storage facilities, and places of public gathering.

### EXAMPLE 13: DETERMINING THE AMOUNT OF ARTIFICIALLY APPLIED CODLEMONE IN ORDER TO CONTROL CYDIA POMONELLA LINNAEUS POPULATIONS.

The present invention describes methods to quantify the amount of artificially applied pheromone in the field. This is particularly useful when attempting to control insect pest populations using mating disruption based on pheromone application. After an initial pheromone application, the present invention can indicate when another application is desirable. In this example, the present invention is used to monitor the application of codling moth codlemone.

The gene(s) encoding GPCR(s) responsible for codlemone¹⁰² detection in the codling moth is transformed into *Drosophila,* mammalian, yeast, or other eukaryotic cells incorporating a reporter gene cascade coupled to an ion channel. These cells are then embedded into silica gel capable of transmitting detectable current across two electrodes. If codling moth codlemone is present in the atmosphere, the GPCRs in the cells embedded in the silica gel bind to it, initiating the signaling cascade that results in ion flux (for example, calcium ions) across the plasma membrane. This ion flux causes a change in electrical current or potential that can be measured. In this manner, the present invention can be used to indicate whether further codlemone application is desirable.

Alternatively, an enzymatic reaction resulting in a color change on a strip can be used instead of ion flux for detecting codlemone. In this method, the chemosensory protein is coupled to an enzymatic reporter cascade which, when evoked, releases a colored product (for example, beta.-galactosidase¹⁰⁵). The appearance of this product on the device indicates the presence of codlemone.

### EXAMPLE 14: USING CHEMOSENSORY PROTEINS OR THEIR PEPTIDE DERIVATIVES TO DEVELOP GELS THAT CAN SELECTIVELY REMOVE ODORS FROM THE ENVIRONMENT.

The present invention describes the compositions and methods desirable to develop highly effective products to inhibit odors. These products can be used in any environment where unpleasant odors need to be contained. To develop these products, odorant-binding proteins can be purified, enriched or identified using the methods provided by the present invention.

For example, DNA sequences encoding OBPs or peptide derivatives are expressed *in vitro* using tools common in the art. The OBPs or peptide derivatives are then incorporated into aqueous gels that can be deployed in environments where odor control is desirable. Odors, scents, or semiochemicals emanating from those environments will be bound by the OBP, or peptide derivatives of the OBP, that essentially act as odor traps. Detectable odors will thus be greatly reduced. These odor control gels incorporating OBPs or OBP peptide derivatives are useful in locations including but not limited to private or public lavatories, garages, kitchens, storage areas, or refuse containers.

### EXAMPLE 15: USING CHEMOSENSORY PROTEINS EXPRESSED IN BACTERIAL CELLS TO PURITY WATER.

Water purification or treatment plants focus on removing pollutants, waste, and other undesirable compounds from water collected in drain systems in order to make the water useable for a variety of applications, or simply to make the water safe enough to dispose of into rivers, lakes, or seas without contaminating the environment. For example, volatile organic compounds are highly undesirable in water, and treatment plants must purify the water of these compounds.

Undesirable volatile organic compounds (VOCs) are screened using an insect chemosensory protein array in order to identify those chemosensory proteins that are capable of recognizing the VOCs as described previously. DNA sequences encoding those chemosensory proteins are then cloned into bacterial expression and transformation vectors common in the art in order to transform E. coli bacteria. The bacterial cells will express the recombinant insect chemosensory proteins and incorporate the proteins into the cell membrane such that the proteins are present on the external cell surfaces. Cultures of these bacteria can be grown in water to be treated; the chemosensory proteins on the bacterial cell surfaces will bind to the undesirable VOCs and remove them from the solution. Thus, this aspect of the invention is suitable for use in water treatment plants.

### REFERENCES

All headings are for the convenience of the reader and should not be used to limit the meaning of the text that follows the heading, unless so specified.
1. Canhoto, O. F. & Magan, N. Potential for detection of microorganisms and heavy metals in potable water using electronic nose technology. Biosens Bioelectron 18, 751-4 (2003).
2. Ouellette, J. Electronic noses sniff out new markets. The Industrial Physicist 5 (1999).
3. Harper, W. J. The strengths and weaknesses of the electronic nose. Adv Exp Med Biol 488, 59-71 (2001).
4. Haugen, J. E. Electronic noses in food analysis. Adv Exp Med Biol 488, 43-57 (2001).
5. Pavlou, A.K. & Turner, A. P. Sniffing out the truth: clinical diagnosis using the electronic nose. Clin Chem Lab Med 38, 99-112 (2000).
6. Phillips, M. et al. Volatile markers of breast cancer in the breath. Breast J 9, 184-91 (2003).
7. Phillips, M., Cataneo, R. N., Greenberg, J., Grodman, R. & Salazar, M. Breath markers of oxidative stress in patients with unstable angina. Heart Dis 5, 95-9 (2003).
8. Deng, C., Zhang, X. & Li, N. Investigation of volatile biomarkers in lung cancer blood using solid-phase microextraction and capillary gas chromatography-mass spectrometry. J Chromatogr B Analyt Technol Biomed Life Sci 808, 269-77 (2004).
9. Phillips, M., Cataneo, R. N., Cheema, T. & Greenberg, J. Increased breath biomarkers of oxidative stress in diabetes mellitus. Clin Chim Acta 344, 189-94 (2004).
10. Thaler, E. R. & Hanson, C. W. Medical applications of electronic nose technology. Expert Rev Med Devices 2, 559-66 (2005).
11. Shnayderman, M. et al. Species-specific bacteria identification using differential mobility spectrometry and bioinformatics pattern recognition. Anal Chem 77, 5930-7 (2005).
12. Poli, D. et al. Exhaled volatile organic compounds in patients with non-small cell lung cancer: cross sectional and nested short-term follow-up study. Respir Res 6, 71 (2005).
13. Barker, M. et al. Volatile organic compounds in the exhaled breath of young patients with cystic fibrosis. Eur Respir J (2006).
14. Lundstrom, I. Artificial 'olfactory' images from a chemical sensor using a light-pulse technique. Nature 352, 47-50 (1991).
15. Corcoran, P., Shurmer, H. & Gardner, J. Integrated tin oxide sensors of low power consumption for use in gas and odour sensing. Sensors and Actuators B 15:16, 32-37 (1993).
16. Shurmer, H. & Gardner, J. Odour discrimination with an electronic nose. Sensors and Actuators B. 8, 1-11 (1992).
17. Gardner, J., Shurmer, H. & Corcoran, P. Integrated tin oxide odour sensors. Sensors and Actuators B 4, 117-121 (1991).
18. Pearce, T. C., Shiffman, S. S., Nagle, H. T. & Gardner, J. Handbook of Machine Olfaction: Electronic Nose Technology (Wiley-VCH Verlag GmbH & Co. KGaA, 2003).
19. de Lorimier; R. M. et al. Construction of a fluorescent biosensor family. Protein Sci 11, 2655-75 (2002).
20. Alvarez, M. et al. Development of nanomechanical biosensors for detection of the pesticide DDT. Biosens Bioelectron 18, 649-53 (2003).
21. Alocilja, E. C. & Radke, S. M. Market analysis of biosensors for food safety. Biosens Bioelectron 18, 841-6 (2003).
22. Davenport, M. et al. Chemical sensing sytems using Xerogel-based sensor elements and CMOS photodetectors. IEEE Sensors J. 4, 180-188 (2004).
23. Justice, R. W., Biessmann, H., Walter, M. F., Dimitratos, S. D. & Woods, D. F. Genomics spawns novel approaches to mosquito control. Bioessays 25, 1011-20 (2003).
24. Pavlou, A. K. et al. Detection of Mycobacterium tuberculosis (TB) in vitro and in situ using an electronic nose in combination with a neural network system. Biosens Bioelectron 20, 538-44 (2004).
25. Turner, A. P. & Magan, N. Electronic noses and disease diagnostics. Nat Rev Microbiol 2, 161-6 (2004).
26. Biessmann, H., Walter, M. F., Dimitratos, S. & Woods, D. Isolation of cDNA clones encoding putative odourant binding proteins from the antennae of the malaria-transmitting mosquito, Anopheles gambiae. Insect Mol Biol 11, 123-32 (2002).
27. Justice, R. W., Dimitratos, S., Walter, M. F., Woods, D. F. & Biessmann, H. Sexual dimorphic expression of putative antennal carrier protein genes in the malaria vector Anopheles gambiae. Insect Mol Biol 12, 581-94 (2003).
28. Biessmann, H., Nguyen, Q. K., Le, D. & Walter, M. F. Microarray-based survey of a subset of putative olfactory genes in the mosquito Anopheles gambiae. Insect Mol Biol 14, 575-89 (2005).
29. Gullan, P. J. & Cranston, P. S. The Insects: An Outline of Entomology (Chapman & Hall, London, 1994).
30. Sambrook, J., Fritsch, E. F. & Maniatis, T. Molecular Cloning: A laboratory manual (ed. Nolan, C.) (Corld Spring Harbor Laboratory Press, Cold Spring Harbor, 1989).
31. Briand, L. et al. Isotopic double-labeling of two honeybee odorant-binding proteins secreted by the methylotrophic yeast Pichia pastoris. Protein Expr Purif 23, 167-74 (2001).
32. Briand, L., Nespoulous, C., Huet, J. C., Takahashi, M. & Pernollet, J. C. Ligand binding and physico-chemical properties of ASP2, a recombinant odorant-binding protein from honeybee (Apis mellifera L.). Eur J Biochem 268, 752-60 (2001).
33. Briand, L., Nespoulous, C., Huet, J. G. & Pernollet, J. C. Disulfide pairing and secondary structure of ASP1, an olfactory-binding protein from honeybee (Apis mellifera L). J Pept Res 58, 540-5 (2001).
34. Briand, L. et al. Characterization of a chemosensory protein (ASP3c) from honeybee (Apis mellifera L.) as a brood pheromone carrier. Eur J Biochem 269, 4586-96 (2002).
35. Backman, A. C. et al. Antennal response of codling moth males, Cydia pomonella L. (Lepidoptera: Tortricidae), to the geometric isomers of codlemone and codlemone acetate. J Comp Physiol [A] 186, 513-9 (2000).
36. Ban, L., Zhang, L., Yan, Y. & Pelosi, P. Binding properties of a locust's chemosensory protein. Biochem Biophys Res Commun 293, 50-4 (2002).
37. Calvello, M. et al. Soluble proteins of chemical communication in the social wasp Polistes dominulus. Cell Mol Life Sci 60, 1933-43 (2003).
38. Pelosi, P. Odorant-binding proteins: structural aspects. Ann N YAcad Sci 855, 281-93 (1998).
39. Scaloni, A., Monti, M., Angeli, S. & Pelosi, P. Structural analysis and disulfide-bridge pairing of two odorant-binding proteins from Bombyx mori. Biochem Biophys Res Commun 266, 386-91 (1999).
40. Hekmat-Scafe, D. S., Scafe, C. R., McKinney, A. J. & Tanouye, M. A. Genome-wide analysis of the odorant-binding protein gene family in Drosophila melanogaster. Genome Res 12, 1357-69 (2002).
41. Vogt, R. G., Rogers, M. E., Franco, M. D. & Sun, M. A comparative study of odorant binding protein genes: differential expression of the PBP1-GOBP2 gene cluster in Manduca sexta (Lepidoptera) and the organization of OBP genes in Drosophila melanogaster (Diptera). J Exp Biol 205, 719-44 (2002).
42. Deyu, Z. & Leal, W. S. Conformational isomers of insect odorant-binding proteins. Arch Biochem Biophys 397, 99-105 (2002).
43. Jin, X. et al. Expression and immunolocalisation of odorant-binding and chemosensory proteins in locusts. Cell Mol Life Sci 62, 1156-66 (2005).
44. Plettner, E., Lazar, J., Prestwich, E. G. & Prestwich, G. D. Discrimination of pheromone enantiomers by two pheromone binding proteins from the gypsy moth Lymantria dispar. Biochemistry 39, 8953-62 (2000).
45. Pelosi, P. & Maida, R. Odorant-binding proteins in insects. Comp Biochem Physiol B Biochem Mol Biol 111, 503-14 (1995).
46. Steinbrecht, R. A. Odorant-binding proteins: expression and function. Ann N Y Acad Sci 855, 323-32 (1998).
47. Looger, L. L., Dwyer, M. A., Smith, J. J. & Hellinga, H. W. Computational design of receptor and sensor proteins with novel functions. Nature 423, 185-90 (2003).
48. Dwyer, M. A., Looger, L. L. & Hellinga, H. W. Computational design of a biologically active enzyme. Science 304, 1967-71 (2004).
49. Xiong, A. S. et al. A simple, rapid, high-fidelity and cost-effective PCR-based two-step DNA synthesis method for long gene sequences. Nucleic Acids Res 32, e98 (2004).
50. Rodacy, P. J., Bender, S., Bromenshenk, J., Henderson, C. & Bender, G. Deployment of honeybees to detect explosives and other agents of harm. Proc. SPIE 4743, 474-481 (2002).
51. Barasic, D., Bromenshenk, J. J., Kezic, N. & Vertacnik, A. in Honey Bees: Estimating the Environmental Impact of Chemicals (eds. Devillers, J. & Pham-Delegue, M. H.) 160-185 (Taylor and Francis, New York, 2002).
52. Shimek, C. 1-5 (University of Montana - Missoula, Missoula, 2004).
53. Takken, W. & Knols, B. G. Odor-mediated behavior of Afrotropical malaria mosquitoes. Annu Rev Entomol 44, 131-57 (1999).
54. Regnier, F. E. & Law, J. H. Insect pheromones. J Lipid Res 9, 541-51 (1968).
55. Renou, M. & Guerrero, A. Insect parapheromones in olfaction research and semiochemical-based pest control strategies. Annu Rev Entomol 45, 605-30 (2000).
56. D'Ettorre, P., Mondy, N., Lenoir, A. & Errard, C. Blending in with the crowd: social parasites integrate into their host colonies using a flexible chemical signature. Proc R Soc Lond B Biol Sci 269, 1911-8 (2002).
57. Ozaki, M. et al. Ant nestmate and non-nestmate discrimination by a chemosensory sensillum. Science 309, 311-4 (2005).
58. Blum, M. S. Semiochemical parsimony in the Arthropoda. Annu Rev Entomol 41, 353-74 (1996).
59. da Silva Torres, C. S. A., Matthews, R. W., Ruberson, J. R. & Lewis, W. J. Role of chemical cues and natal rearing effect on host recognition by the parasitic wasp Melittobia digitata. Entomological Science 8, 355-367 (2005).
60. Mattiacci, L., Hütter, E. & Dom, S. Host location of Hyssopus pallidus, a larval parasitoid of the codling moth, Cydia pomonella. Biological Control 15, 241-251 (1999).
61. Yu, F., Persson, B., Lofas, S. & Knoll, W. Surface plasmon fluorescence immunoassay of free prostate-specific antigen in human plasma at the femtomolar level. Anal Chem 76, 6765-70 (2004).
62. Fradet, Y. et al. uPM3, a new molecular urine test for the detection of prostate cancer. Urology 64, 311-5; discussion 315-6 (2004).
63. Saad, F. UPM3: review of a new molecular diagnostic urine test for prostate cancer. Can J Urol 12 Suppl 1, 40-3; discussion 99-100 (2005).
64. Mutlu, N., Turkeri, L. & Emerk, K. Analytical and clinical evaluation of a new urinary tumor marker: bladder tumor fibronectin in diagnosis and follow-up of bladder cancer. Clin Chem Lab Med 41, 1069-74 (2003).
65. Phillips, M. et al. Volatile markers of pulmonary tuberculosis in the breath. Eur Respir J 24, 467s (2004).
66. Phillips, M. et al. Volatile organic compounds in breath as markers of lung cancer: a cross-sectional study. Lancet 353, 1930-3 (1999).
67. Phillips, M. et al. Detection of lung cancer with volatile markers in the breath. Chest 123, 2115-23 (2003).
68. Pickel, D., Manucy, G. P., Walker, D. B., Hall, S. B. & Walker, J. C. Evidence for canine olfactory detection of melanoma. Applied Animal Behavior Science 89, 107-116 (2004).
69. Dalton, P., Gelperin, A. & Preti, G. Volatile metabolic monitoring of glycemic status in diabetes using electronic olfaction. Diabetes Technol Ther 6, 534-44 (2004).
70. Sieg, A., Guy, R. H. & Delgado-Charro, M. B. Noninvasive and minimally invasive methods for transdermal glucose monitoring. Diabetes Technol Ther 7, 174-97 (2005).
71. Newman, J. D. & Turner, A. P. Home blood glucose biosensors: a commercial perspective. Biosens Bioelectron 20, 2435-53 (2005).
72. Schutz, S. et al. Insect antenna as a smoke detector. Nature 398, 298-299 (1999).
73. Wright, R. H. Why mosquito repellents repel. Sci Am 233, 104-11 (1975).
74. Dogan, E. B., Ayres, J. W. & Rossignol, P. A. Behavioural mode of action of deet: inhibition of lactic acid attraction. Med Vet Entomol 13, 97-100 (1999).
75. Bohbot, J., Sobrio, F., Lucas, P. & Nagnan-Le Meillour, P. Functional characterization of a new class of odorant-binding proteins in the moth Mamestra brassicae. Biochem Biophys Res Commun 253, 489-94 (1998).
76. Callahan, F. E., Vogt, R. G., Tucker, M. L., Dickens, J. C. & Mattoo, A. K. High level expression of "male specific" pheromone binding proteins (PBPs) in the: antennae of female noctuiid moths. Insect:Biochem Mol Biol 30, 507-14 (2000).
77. Dickens, J. C., Callahan, F. E., Wergin, W. P., Murphy, C. A. & Vogt, R. G. Odorant-binding proteins of true bugs. Generic specificity, sexual dimorphism, and association with subsets of chemosensory sensilla. Ann N YAcad Sci 855, 306-10 (1998).
78. Du, G. & Prestwich, G. D. Protein structure encodes the ligand binding specificity in pheromone binding proteins. Biochemistry 34, 8726-32 (1995).
79. Gyorgyi, T. K., Roby-Shemkovitz, A. J. & Lerner, M. R. Characterization and cDNA cloning of the pheromone-binding protein from the tobacco hornworm, Manduca sexta: a tissue-specific developmentally regulated protein. Proc Natl Acad Sci USA 85, 9851-5 (1988).
80. Hildebrand, J. G. King Solomon Lecture - Olfactory control of behavior in moths: central processing of odor information and the functional significance of olfactory glomeruli. J. Comp. Physiol. A, 5-19 (1996).
81. Ishida, Y., Cornel, A. J. & Leal, W. S. Identification and cloning of a female antenna-specific odorant-binding protein in the mosquito Culex quinquefasciatus. J Chem Ecol 28, 867-71 (2002).
82. Jacobson, M. & Jones, W. A. Attraction of the male pink bollworm moth under laboratory and field conditions. Environ Lett 6, 297-301 (1974).
83. Jacquin-Joly, E., Bohbot, J., Francois, M. C., Cain, A. H. & Nagnan-Le Meillour, P. Characterization of the general odorant-binding protein 2 in the molecular coding of odorants in Mamestra brassicae. Eur J Biochem 267, 6708-14 (2000).
84. Jacquin-Joly, E., Vogt, R. G., Francois, M. C. & Nagnan-Le Meillour, P. Functional and expression pattern analysis of chemosensory proteins expressed in antennae and pheromonal gland of Mamestra brassicae. Chem Senses 26, 833-44 (2001).
85. Jones, W. A. & Jacobson, M. Isolation of N,N-diethyl-m-toluamide (deet) from female pink bollworm moths. Science 159, 99-100 (1968).
86. Kaissling, K. E. Olfactory perireceptor and receptor events in moths: a kinetic model. Chem Senses 26, 125-50 (2001).
87. Krieger, J., Ganssle, H., Raming, K. & Breer, H. Odorant binding proteins of Heliothis virescens. Insect Biochem Mol Biol 23, 449-56 (1993).
88. Krieger, J., von Nickisch-Rosenegk, E., Mameli, M., Pelosi, P. & Breer, H. Binding proteins from the antennae of Bombyx mori. Insect Biochem Mol Biol 26, 297-307 (1996).
89. Leal, W. S., Wojtasek, H. & Miyazawa, M. Pheromone-binding proteins of scarab beetles. Ann N Y Acad Sci 855, 301-5 (1998).
90. Field, L. M., Pickett, J. A. & Wadhams, L. J. Molecular studies in insect olfaction: Insect Mol Biol 9, 545-51 (2000).
91. Krieger, J. & Breer, H. Olfactory reception in invertebrates. Science 286, 720-3 (1999).
92. Mombaerts, P. Molecular biology of odorant receptors in vertebrates. Annu Rev Neurosci 22, 487-509 (1999).
93. Blenau, W., Erber, J. & Baumann, A. Characterization of a dopamine D1 receptor from Apis mellifera: cloning, functional expression, pharmacology, and mRNA localization in the brain. J Neurochem 70, 15-23 (1998).
94. Briand, L. et al. Ligand-binding properties and structural characterization of a novel rat odorant-binding protein variant. Eur JBiochem 267, 3079-89 (2000).
95. Deglise, P., Grunewald, B. & Gauthier, M. The insecticide imidacloprid is a partial agonist of the nicotinic receptor of honeybee Kenyon cells. Neurosci Lett 321, 13-6 (2002).
96. Wojtasek, H. & Leal, W. S. Conformational change in the pheromone-binding protein from Bombyx mori induced by pH and by interaction with membranes. J. Biol. Chem. 274, 30950-30956 (1999).
97. Bromenshenk, J., Seccomb, R. A., Rice, S. D., Etter, R. T. & Henderson, C. B. in United States Patent Office (The University of Montana (Missoula, MT), USA, 2003).
98. Costantini, C., Sagnon, N., della Torre, A. & Coluzzi, M. Mosquito behavioural aspects of vector-human interactions in the Anopheles gambiae complex. Parassitologia 41, 209-17 (1999).
99. Dekker, T., Steib, B., Carde, R. T. & Geier, M. L-lactic acid: a human-signifying host cue for the anthropophilic mosquito Anopheles gambiae. Med. Vet. Entomol. 16, 91-98 (2002).
100. Hallem, E. A., Nicole Fox, A., Zwiebel, L. J. & Carlson, J. R. Olfaction: mosquito receptor for human-sweat odorant. Nature 427, 212-3 (2004).
101. Brunner, J. et al. in IOBC wprs Bulletin Vol. 25 (2002).
102. Knight, A. Managing codling moth (Lepidoptera:Tortricidae) with an internal grid of either aerosol puffers or dispenser clusters plus border applications of individual dispensers. J. Entomol. Soc. Brit. Columbia 101, 69-78 (2004).
103. Knight, A. L. Monitoring codling moth (Lepidoptera: Tortricidae) with passive interception traps in sex pheromone-treated apple orchards. JEcon Entomol 93, 1744-51 (2000).
104. Knight, A. L., Dunley, J. E. & Jansson, R. K. Baseline monitoring of codling moth (Lepidoptera: Tortricidae) larval response to benzoylhydrazine insecticides. J Econ Entomol 94, 264-70 (2001).
105. Candido, E. P. & Jones, D. Transgenic Caenorhabditis elegans strains as biosensors. Trends Biotechnol 14, 125-9 (1996).
106. Wu, T. Z. A piezoelectric biosensor as an olfactory receptor for odour detection: electronic nose. Biosens Bioelectron 14, 9-18 (1999).
107. Tombelli, S., Minunni, M. & Mascini, M. Piezoelectric biosensors: strategies for coupling nucleic acids to piezoelectric devices. Methods 37, 48-56 (2005).

## Claims

1. A method of identifying chemicals in the atmosphere or in solution using a biosensor, comprising:
a) providing isolated chemosensory proteins or fragments thereof, capable of binding said chemicals of interest, said proteins or fragments being sourced from different insect species ;
b) incorporating said proteins or fragments into a reporter system; and
c) detecting activation of said reporter system, whereby the presence of said chemicals is detected,
wherein said proteins or fragments comprise odorant-binding protein, sensory appendage protein, ortholog of *Drosophila* melanogaster Takeout protein, and odorant degrading enzymes.

2. A method according to claim 1, wherein said chemicals are semiochemicals.

3. A method of generating a chemosensory "profile" or "fingerprint" for a given analyte comprising:
a) generating a collection of isolated invertebrate chemosensory proteins or fragments thereof, said proteins or fragments being sourced from different insect species ;
b) linking said collection to a reporter mechanism, such as an enzymatic or fluorescence based reporter mechanism, to generate a chemosensory protein array; and
c) exposing the analyte to the chemosensory array and recording the identities of the chemosensory proteins or fragments thereof in the array capable of binding the analyte, whereupon a chemosensory fingerprint of the analyte is generated,
wherein said proteins or fragments comprise odorant-binding protein, sensory appendage protein, ortholog of *Drosophila* melanogaster Takeout protein, and odorant degrading enzymes.

4. The method of claim 1 or 3, wherein subsets of the chemosensory array containing chemosensory proteins or fragments thereof, capable of binding a specific analyte or a complex mixture of analytes, are used to generate a detection or purification device for the particular analyte or the complex mixture of analytes.

5. The method of claim 1 or 3, wherein said reporter system causes an electrical change that can be measured, is activated enzymatically, is activated chemically, is activated electrically, is based on surface plasmon resonance, uses a cell-based assay, or uses a fluorescence-based assay.

6. The method of claim 3, wherein said protein comprises hybrid domains or motifs generated from fragments of recombinant chemosensory proteins combined in a novel manner, or in vitro using directed mutagenesis or recombinant DNA technology.

7. A method of purifying natural or synthetic chemicals or analytes in the atmosphere or in solution, comprising:
a) providing isolated chemosensory proteins or fragments thereof, capable of recognizing said analyte of interest, said proteins or fragments being sourced from different insect species ; and
b) incorporating said chemosensory protein or fragment into a selective filtration device, wherein said proteins or fragments comprise odorant-binding protein, sensory appendage protein, ortholog of *Drosophila* melanogaster Takeout protein, and odorant degrading enzymes.

## Patentansprüche

1. Ein Verfahren zur Identifizierung von Chemikalien in Atmosphäre oder Lösung unter Verwendung eines Biosensors umfassend :
a) Bereitstellung von isolierten chemosensorischen Proteinen oder Fragmenten davon, die fähig sind, die Chemikalien von Interesse zu binden, wobei die Proteine oder Fragmente von verschiedenen Insektenspezies stammen;
b) Einbringen der Proteine oder Fragmente in ein Reporter-System; und
c) Nachweis der Aktivierung des Reporter-Systems, wobei das Vorhandensein der Chemikalien nachgewiesen wird,
wobei die Proteine oder Fragmente Geruchsstoff-bindendes Protein, sensorisches Anhängselprotein, Orthologe des *Drosophila melanogaster* Takeout-Proteins, und Geruchsstoff-abbauende Enzyme umfassen.

2. Ein Verfahren nach Anspruch 1, wobei die Chemikalien Semiochemikalien sind.

3. Ein Verfahren zur Erstellung eines chemosensorischen "Profils" oder "Fingerabdrucks" für ein gegebenes Analyt umfassend:
a) Erstellung einer Bibliothek von isolierten, chemosensorischen Invertebratenproteinen oder Fragmenten davon, wobei die Proteine von verschiedenen Insektenspezies stammen;
b) Verbindung der Bibliothek mit einem Reporter-Mechanismus, wie zum Beispiel einem Reporter-Mechanismus auf enzymatischer oder Fluoreszenz-Basis zur Erzeugung eines chemosensorischen Protein-Arrays; und
c) Exposition des Analyten in dem chemosensorischen Array und Aufzeichnen der Identitäten der chemosensorischen Proteine oder Fragmente davon in dem Array, das den Analyten binden kann, worauf ein chemosensorischer Fingerabdruck des Analyten erzeugt wird,
wobei besagte Proteine oder Fragmente Geruchsstoff-bindendes Protein, sensorisches Anhängselprotein, Orthologe des *Drosophila melanogaster* Takeout-Proteins, und Geruchsstoff-abbauende Enzyme umfassen.

4. Verfahren nach Anspruch 1 oder 3, wobei Untereinheiten des chemosensorischen Arrays, die chemosensorische Proteine oder Fragmente davon enthalten, die fähig sind, einen spezifischen Analyten oder eine komplexe Mischung von Analyten zu binden, zur Herstellung einer Nachweis- oder Reinigungsvorrichtung für den bestimmten Analyten oder die komplexe Mischung von Analyten verwendet werden.

5. Verfahren nach Anspruch 1 oder 3, wobei das eine messbare elektrische Veränderung hervorrufende Reporter-System enzymatisch aktiviert wird, chemikalisch aktiviert wird, elektrisch aktiviert wird, auf einer Oberflächenplasmon-Resonanz basiert ist, einen Zell-basierten Assay verwendet oder einen Fluoreszenzbasierten Assay verwendet.

6. Verfahren nach Anspruch 3, wobei das Protein Hybriddomänen oder Motive umfasst, die von neuartig kombinierten Fragmenten rekombinanter chemosensorischer Proteine oder in vitro unter Verwendung der Technologie der rekombinanten DNA oder der gesteuerten Mutagenese erzeugt werden.

7. Ein Verfahren zur Reinigung natürlicher oder synthetischer Chemikalien oder Analyten in Atmosphäre oder in Lösung, umfassend:
a) Bereitstellung von isolierten chemosensorischen Proteinen oder Fragmenten davon, die fähig sind, Analyten von Interesse zu erkennen, wobei die Proteine oder Fragmente von verschiedenen Insektenspezies stammen; und
b) Einbringen der chemosensorischen Proteine oder Fragmente in eine selektive Filtriervorrichtung, wobei die Proteine oder Fragmente Geruchsstoff-bindendes Protein, sensorisches Anhängselprotein, Orthologe des *Drosophila melanogaster* Takeout-Proteins, und Geruchsstoff-abbauende Enzyme umfassen.

## Revendications

1. Procédé d'identification de produits chimiques dans l'atmoshère ou en solution utilisant un biocapteur, comprenant :
a) la fourniture de protéines chimiosensorielles isolées ou de fragments de celles-ci, capables de lier lesdits produits chimiques d'intérêt, lesdites protéines ou fragments provenant de différentes espèces d'insectes ;
b) l'incorporation desdites protéines ou fragments dans un système rapporteur ; et
c) la détection de l'activation dudit système rapporteur, de sorte que la présence desdits produits chimiques est détectée,
dans lequel lesdites protéines ou fragments comprennent des protéines liant les odeurs, des protéines d'appendices sensoriels, des orthologues de la protéine Takeout de *Drosophila* melanogaster, et des enzymes dégradant les odeurs.

2. Procédé selon la revendication 1, dans lequel lesdits produits chimiques sont des sémiochimiques.

3. Procédé de génération d'un « profil » ou d'une « empreinte » chimiosensoriel pour un analyte donné comprenant :
a) la génération d'une collection de protéines chimiosensorielles d'invertébrés isolées ou fragments de celles-ci, lesdites protéines ou fragments provenant de différentes espèces d'insectes ;
b) la liaison de ladite collection à un mécanisme rapporteur, tel qu'un mécanisme rapporteur à base enzymatique ou de fluorescence, afin de générer un ensemble de protéines chimiosensorielles ; et
c) l'exposition de l'analyte à l'ensemble chimiosensoriel et l'enregistrement des identités des protéines chimiosensorielles ou fragments de celles-ci dans l'ensemble capables de lier l'analyte, après quoi une empreinte chimiosensorielle de l'analyte est générée,
dans lequel lesdites protéines ou fragments comprennent des protéines liant les odeurs, des protéines d'appendices sensoriels, des orthologues de la protéine Takeout de *Drosophila* melanogaster, et des enzymes dégradant les odeurs.

4. Procédé selon la revendication 1 ou 3, dans lequel des sous-ensembles de l'ensemble chimiosensoriel contenant des protéines chimiosensorielles ou des fragments de celles-ci, capables de lier un analyte spécifique ou un mélange complexe d'analytes, sont utilisés afin de générer un dispositif de détection ou de purification pour l'analyte particulier ou le mélange complexe d'analytes.

5. Procédé selon la revendication 1 ou 3, dans lequel ledit système rapporteur provoque un changement électrique qui peut être mesuré, est activé de manière enzymatique, est activé de manière chimique, est activé de manière électrique, est basé sur la résonance plasmonique de surface, utilise un réseau à base de cellules, ou utilise un réseau basé sur la fluorescence.

6. Procédé selon la revendication 3, dans lequel ladite protéine comprend des motifs ou domaines hybrides générés à partir de fragments de protéines chimiosensorielles recombinantes combinés d'une nouvelle manière, ou utilisant *in vitro* la mutagenèse dirigée ou la technologie de l'ADN recombinant.

7. Procédé de purification de produits chimiques ou d'analytes naturels ou synthétiques dans l'atmosphère ou en solution, comprenant :
a) la fourniture de protéines chimiosensorielles isolées ou fragments de celles-ci, capables de reconnaitre ledit analyte d'intérêt, lesdites protéines ou fragments provenant de différentes espèces d'insectes ; et
b) l'incorporation desdites protéines chimiosensorielles ou fragments dans un dispositif de filtration sélectif, dans lequel lesdites protéines ou fragments comprennent des protéines liant les odeurs, des protéines d'appendices sensoriels, des orthologues de la protéine Takeout de *Drosophila* melanogaster, et des enzymes dégradant les odeurs.
